# EUROPEAN PATENT APPLICATION

(11) **EP 3 056 197 A1**
(43) Date of publication of application: **17.08.2016**
(21) Application number: 16150552.4
(22) Date of filing: 19.11.2010
(51) Int. Cl.: A61K 9/50, A61K 38/47, A61K 38/46, A61K 45/06, A61K 38/48, A61K 9/20

(54) **PREPARATION AND USE OF COMBINATION ENZYME AND GASTROINTESTINAL MODULATOR DELIVERY SYSTEMS**

(62) Divisional of application: 10859701.4
(71) Applicant: Curemark, LLC, Rye, NY 10580 (US)
(72) Inventor: FALLON, Joan M., Bronxville, NY New York 10708 (US); HEIL, Matthew, Sherman, CT Connecticut 06784 (US); SZIGETHY, James F., Montgomery, NY New York 12549 (US); FALLON, James, Armonk, NY New York 10504 (US)
(74) Representative: Patent Boutique LLP

(57) **Abstract**

Pharmaceutical compositions comprising one or more digestive enzymes and one or more gastrointestinal modulators of acid are provided. The one or more digestive enzymes may be coated, e.g., with a lipid. Also disclosed are methods for their use and controlled delivery in treating individuals with neurological, behavioural, infectious, or genetic diseases or conditions susceptible to treatment with digestive enzymes.

## Description

### FIELD OF THE INVENTION

The present disclosure relates generally to compositions comprising one or more digestive enzymes and one or more gastrointestinal modulators of acid, e.g., stomach acid, along with pharmaceutical compositions and enzyme delivery systems comprising the same, as well as methods for their use and controlled delivery in treating individuals with neurological, behavioral, infectious, or genetic diseases or conditions susceptible to treatment with digestive enzymes.

The following Patents and Patents Pending are fully incorporated by reference: US 09/707,395 filed November 7, 2000 issued on October 14, 2003 as US 6,632, 429 B1, Methods for Treating Pervasive Development Disorders; US 11/555,697 filed November 11, 2006, Methods for Treating and Diagnosing Parkinson's Disease and Related Dysautonomia Disorders; US 11/533,818 filed on September 21, 2006, Pharmaceutical Preparations for Attention Deficit Disorder, Attention Deficit Hyper Activity Disorder and Other Associated Disorders; US 12/386,051 filed April 13, 2009 Enzyme Delivery Systems and Methods of Preparation and Use; US 12/493,147 filed June 26, 2009 Pharmaceutical Preparation for Complex Regional Pain Syndrome and Method of Making Same; PCT/US09/49374 filed July 1, 2009, Pharmaceutical Preparation for Treatment of Neurological and Mental Health Disorders and Method of Making Same; US 12/426,794 filed April 20, 2009, Pancreatic Enzymes in the Treatment of Addiction; US 12/493,122 filed June 26, 2009, Pharmaceutical Preparation for Treatment of William's Syndrome and Method of Making Same; US 11/232,180 filed October 2, 2008, Combination Enzyme for Cystic Fibrosis; and US 61/102,818 filed October 3, 2008 filed Pharmaceutical Preparation for Treatment of Prion Disease and Method of Making Same, and US 61/253,805 filed October 21, 2009, Methods and Compositions for the Prevention and Treatment of Influenza.

### BACKGROUND

Digestive enzymes are enzymes that can break down one or more components of foods, e.g., carbohydrates, lipids/fats, proteins, cellulose, nucleic acids, etc., and thereby assist in digestion and uptake of nutrients. Certain digestive enzymes are produced by the salivary glands, glands in the stomach, the pancreas, and glands in the small intestines. For example, digestive enzymes produced by the pancreas and secreted into the stomach and small intestine aid in digestion. Other digestive enzymes are produced by plants, fungi, or microorganisms (e.g. papain, bromelain).

Digestive enzymes produced by the pancreas and secreted into the stomach and small intestine aid food enzymes in digestion. Digestive enzymes produced by the pancreas are secreted into the duodenum, or upper segment of the small intestine, where the pH is around 5 or 6, and the enzymes assist in the digestion of food components, including carbohydrates, lipids, and proteins.

Digestive enzymes have been administered to mammals to treat enzyme deficiencies caused by conditions affecting the pancreas, such as pancreatitis and pancreatic enzyme deficiency. Pancreatic enzymes administered to humans are commonly of porcine origin. Manufacturers of enzyme preparations have also used enteric coatings for lipase compositions in individuals with cystic fibrosis who require administration of lipases. The preparations for lipase delivery have used enteric coatings containing, for example, hypromellose phthalate, dimethicone 1000, and dibutyl phthalate.

Certain methods for coating sensitive bioactive substances such as enzymes have been described, *see, e.g.,* US RE40,059; 6,835,397; 6,797,291; 6,616,954; 6,312,741; 6,251,478; 6,153,236; 6,013,286, and 5,190,775, and Ser. No. 12/386,051, all ofwhich are incorporated by reference in their entirety herein.

No description in the Background section should be taken as an admission that such disclosure constitutes prior art to the instant disclosure.

### SUMMARY OF THE DISCLOSURE

The present disclosure relates to the use of gastrointestinal modulators of stomach acid utilized in combination with coated or uncoated digestive enzyme compositions (digestive enzyme compositions are also referred to as PEC herein) and pharmaceutical compositions thereof which are useful in the treatment of individuals with autism, ADD, ADHD, Parkinson's disease, cystic fibrosis and other neurological or behavioral diseases or conditions. The combination of gastrointestinal modulators of stomach acid (also referred to as stomach acid reducers herein) and uncoated or coated digestive enzyme preparations of this disclosure enhance the controlled delivery of enzymes having increased stability and enhanced administration properties, to patients with neurological, behavioral, infectious, or genetic diseases and conditions susceptible to treatment with digestive enzymes.

In some aspects, the present disclosure also relates to such combinations which comprise a coated digestive enzyme preparation which comprises a core comprising one or more digestive (e.g., pancreatic) enzymes and a coating which comprises an emulsifiable lipid. The core contains an amount of digestive enzymes effective for treatment of the patient's condition, which can be, for example, a neurological disorder such as autism, ADD, ADHD, CF and Parkinson's disease, or other diseases for which an effective amount of digestive enzymes can be administered. Among other properties, the coating protects the digestive enzymes from destabilizing factors such as solvents, heat, light, moisture and other environmental factors. The coating also provides controlled release of the digestive enzymes when exposed to a solvent.

In some aspects, a coated digestive enzyme preparation for use in the disclosed combinations comprises (a) a core containing a digestive enzyme particle, where the enzyme(s) is/are present in an amount of from about 5% to 95% by weight of the particles; and (b) a coating comprising an emulsifiable lipid, wherein the coating continuously coats the core and the emulsifiable lipid emulsifies upon exposure to a solvent.

In another aspect, this disclosure relates to a pharmaceutical composition comprising a therapeutically effective amount of a gastrointestinal modulator of acid and a coated digestive enzyme preparation, which comprises (a) a core which comprises an amount of one or more digestive enzymes effective for treating a subject suffering from autism, ADD, ADHD, Parkinson's' disease, cystic fibrosis, or other neurological condition or behavioral disorder susceptible to treatment by the enzymes; and (b) a coating comprising an emulsifiable lipid.

Autism (sometimes called "classical autism") is the most common condition in a group of developmental disorders known as the autism spectrum disorders (ASDs). Autism is characterized by impaired social interaction, problems with verbal and nonverbal communication, and unusual, repetitive, or severely limited activities and interests. Other ASDs include Asperger syndrome, Rett syndrome, childhood disintegrative disorder, and pervasive developmental disorder not otherwise specified (usually referred to as PDD-NOS). It has been estimated that three to six children out of every 1,000 will have autism.

Attention deficit-hyperactivity disorder (ADHD) is a neurobehavioral disorder that affects 3-5 percent of all children in the US. It interferes with a person's ability to stay on a task and to exercise age-appropriate inhibition (cognitive alone or both cognitive and behavioral). Some of the warning signs of ADHD include failure to listen to instructions, inability to organize oneself and school work, fidgeting with hands and feet, talking too much, leaving projects, chores and homework unfinished, and having trouble paying attention to and responding to details. There are several types of ADHD: a predominantly inattentive subtype, a predominantly hyperactive-impulsive subtype, and a combined subtype. ADHD is usually diagnosed in childhood, although the condition can continue into the adult years.

Parkinson's disease (PD) belongs to a group of conditions called motor system disorders, which are associated with the loss of dopamine-producing brain cells. The four primary symptoms of PD are tremor, or trembling in hands, arms, legs, jaw, and face; rigidity, or stiffness of the limbs and trunk; bradykinesia, or slowness of movement; and postural instability, or impaired balance and coordination. As these symptoms become more pronounced, patients may have difficulty walking, talking, or completing other simple tasks. PD usually affects people over the age of 50. Early symptoms of PD are subtle and occur gradually. In some people the disease progresses more quickly than in others. As the disease progresses, the shaking, or tremor, which affects the majority of PD patients may begin to interfere with daily activities. Other symptoms may include depression and other emotional changes; difficulty in swallowing, chewing, and speaking; urinary problems or constipation; skin problems; and sleep disruptions.

Cystic fibrosis (CF) is one of the most common life-shortening, genetic diseases. In the United States, 1 in 4,000 children are born with CF. It is most common among western European populations; one in twenty-two people of Mediterranean descent are carriers of one gene for CF, making it the most common genetic disease in these populations. CF is caused by a mutation in the gene, cystic fibrosis transmembrane conductance regulator (CFTR). The product of this gene is a chloride ion channel important in creating sweat, digestive juices, and mucus. Although most people without CF have two working copies (alleles) of the CFTR gene, only one is needed to prevent cystic fibrosis. Cystic fibrosis affects the exocrine (mucus) glands of the lungs, liver, pancreas, and intestines, causing progressive disability due to multisystem failure. CF can be characterized by, for example, 1) thick mucus production which results in frequent lung infections; 2) diminished secretion of pancreatic enzymes causing poor growth, greasy stools, and deficiency in fat-soluble vitamins; and 3) infertility in the males due to the condition congenital bilateral absence of the vas deferens. Often, symptoms of CF appear in infancy and childhood. Meconium ileus is a typical finding in newborn babies with CF.

Enzyme preparations with enteric coatings have been used to deliver lipases in individuals requiring administration of lipases to individuals with cystic fibrosis in need of enzyme treatment. In addition, Fallon has described certain methods and enzyme compositions for use in treating children and other individuals, with autism, ADD, ADHD, Parkinson's disease and other neurological diseases or conditions, for example, U.S. Patent Nos. 7,138,123, 6,660,831, 6,632,429, 6,534,063, hereby incorporated by reference as if set forth in full herein.

The nature of the human digestive tract creates challenges for the delivery of digestive enzymes to patients with neurological and behavioral conditions susceptible to treatment with digestive enzymes. Multiple temperature and pH changes over the course of the digestive tract make specific delivery a necessity and a challenge. For instance, pH as low as 1 is encountered in the stomach, but rapidly increases to a more basic pH of 5-6 in the proximal small intestine. For example, generally the pH in the stomach is approximately 1.2, the pH in the duodenum is about 5.0 to 6.0; the pH in the jejunum is about 6.8, and the pH is about 7.2 in the proximal ileum and about 7.5 in the distal ileum. The low pH in the stomach which changes rapidly to a more basic pH of 5-6 in the proximal small intestines, calls for a specific delivery method depending upon where the enzyme is to be delivered.

For example, children with cystic fibrosis whose condition requires administration of lipases, require delivery of the lipases to the latter portion of the small intestine where lipid digestion and absorption take place. In contrast, the inventors have determined that children with autism who need treatment with proteases require delivery of those enzymes to the proximal small intestine where protein digestion and absorption begins.

Delivery of digestive enzymes can also be challenging due to the rapid degradation and denaturing of enzymes at ambient room temperature, as well as the enhanced degradation and denaturing that can occur with high temperature, pressure, humidity and/or exposure to light. Moisture and heat together can quickly destabilize enzymes, reducing their effectiveness, and shortening shelf life, leading to inaccurate dosing. Denaturization or destabilization of the enzymes can reduce their effectiveness by reducing the dose of active enzymes to less than the amount needed for effective treatment. Alternatively, attempting to compensate for the denaturization or destablization by increasing the dose to ensure an effective level of active enzyme, could risk an overdose or overfilling a capsule or other dosage form. To protect and stabilize the digestive enzyme from unfavorable conditions, the digestive enzyme (core) may be coated in a continuous coating containing an emulsifiable lipid.

The coatings in the digestive enzyme preparations create a barrier to degradation and denaturation, and allow more accurate levels of active enzymes to reach the treated individuals. The lipid coating of this invention provides a significant barrier to moisture, heat, humidity and exposure to light by allowing for a physical barrier as well as one that prevents and or reduces hydrolysis. The coated enzyme preparations undergo less hydrolysis as a result of protection from moisture in the environment by the lipid coating. As a result of the present invention, digestive enzymes are provided which can tolerate storage conditions (e.g., moisture, heat, oxygen, etc.) for long periods of time thus enabling extended shelf life. The coating of the enzyme preparation protects the enzyme from the environment and provides emulsification in a solvent without detracting from the abrasion resistance of the coating. The coated enzyme preparations therefore reduce overfilling of the enzyme dosage, and enhance delivery of more accurate doses of the enzyme to individuals with autism, ADD, ADHD Parkinsons's disease, cystic fibrosis and other neurological or behavioral conditions or diseases susceptible to treatment with pancreatic or other digestive enzymes.

In some embodiments, the coatings on the digestive enzyme particle cores are preferably continuous coatings. By "continuous," it is meant that the pancreatic or other digestive enzyme is uniformly protected. The continuous coating fully surrounds the one or more digestive enzymes. The coating provides protection of the digestive enzyme from conditions such as moisture, temperature, and conditions encountered during storage.

In addition, the coating also provides controlled release of the digestive enzyme. The emulsification properties of the coating in a solvent allows for controlled release of the enzyme in the gastrointestinal system, preferably the region of the GI tract where the enzymes are to be utilized. The coating of the composite protects the enzyme from the environment and provides emulsification in a solvent without detracting from the abrasion resistance of the coating. For example, for conditions requiring treatment with proteases, the release of the protease portion of the enzymes may, in many conditions, need to be in the proximal small intestine, thereby necessitating a lipid coating which has a dissolution profile between 30-90 minutes with 80% or greater release. Lower levels of release are still beneficial and may be utilized in some embodiments of the present invention. The dissolution profile may also be about 30, 35, 40, 45, 50, 55, 60, 65, 70, 75, 80, 85 or 90 minutes. Dissolution profiles may be obtained using methods and conditions known to those of skill in the art. For example, dissolution profiles can be determined at various pHs, including pHs 1, 2, 3, 4, 5, 6, 7, 8, 9 and 10.

In some aspects, multiple layers of coatings can be utilized to provide specific time release profiles of the digestive enzyme(s). In addition continuous or spatially localized coatings may be utilized to allow a specific dissolution profile with certain amounts of enzyme being released at target portions of the GI tract.

The use of gastrointestinal modulators in conjunction with one or more coatings for the enzymes can provide for many different dissolution profiles to achieve optimal efficacy and delivery of the digestive enzymes.

In one aspect of the present disclosure coated or uncoated compositions comprising one or more digestive enzymes are utilized in combination with Histamine-2 receptor antagonists (H2-blockers) to enhance the controlled delivery of digestive enzymes to patients with neurological and behavioral diseases and conditions susceptible to treatment with digestive enzymes.

In one aspect of the present disclosure the H2-Blocker ranitidine (tradename Zantac®)) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure the H2-Blocker nizatidine (tradename Axid®) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure the H2-Blocker famotidine (tradename Pepcid®)) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure the H2-Blocker cimetidine (tradename Tagamet®) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure coated or uncoated compositions comprising one or more digestive enzymes are utilized in combination with Proton Pump Inhibitors (PPIs) to enhance the controlled delivery of enzymes to patients with neurological and behavioral diseases and conditions susceptible to treatment with digestive enzymes.

In one aspect of the present disclosure coated or uncoated compositions comprising one or more digestive enzymes are utilized in combination with the Proton Pump Inhibitor omeprazole (tradename Prilosec®)) to enhance the controlled delivery of enzymes to patients with neurological and behavioral diseases and conditions susceptible to treatment with digestive enzymes.

In one aspect of the present disclosure coated or uncoated compositions comprising one or more digestive enzymes are utilized in combination with the Proton Pump Inhibitor esomeprazole (tradename Nexium®) to enhance the controlled delivery of enzymes to patients with neurological and behavioral diseases and conditions susceptible to treatment with digestive enzymes.

In one aspect of the present disclosure coated or uncoated compositions comprising one or more digestive enzymes are utilized in combination with the Proton Pump Inhibitor omeprazole and sodium bicarbonate (tradename Zegerid®) to enhance the controlled delivery of enzymes to patients with neurological and behavioral diseases and conditions susceptible to treatment with digestive enzymes.

In one aspect of the present disclosure coated or uncoated compositions comprising one or more digestive enzymes are utilized in combination with the Proton Pump Inhibitor lansoprazole (tradename Prevacid®) to enhance the controlled delivery of enzymes to patients with neurological and behavioral diseases and conditions susceptible to treatment with digestive enzymes.

In one aspect of the present disclosure coated or uncoated compositions comprising one or more digestive enzymes are utilized in combination with the Proton Pump Inhibitor dexlansoprazole (tradename Dexilant®) to enhance the controlled delivery of enzymes to patients with neurological and behavioral diseases and conditions susceptible to treatment with digestive enzymes.

In one aspect of the present disclosure coated or uncoated compositions comprising one or more digestive enzymes are utilized in combination with the Proton Pump Inhibitor rabeprazole (tradename AcipHex®) to enhance the controlled delivery of enzymes to patients with neurological and behavioral diseases and conditions susceptible to treatment with digestive enzymes.

In one aspect of the present disclosure coated or uncoated compositions comprising one or more digestive enzymes are utilized in combination with the Proton Pump Inhibitor pantoprazole (tradename Protonix®) to enhance the controlled delivery of enzymes to patients with neurological and behavioral diseases and conditions susceptible to treatment with digestive enzymes.

In one aspect of the present disclosure coated or uncoated compositions comprising one or more digestive enzymes are utilized in combination with Mucosal Protectants to enhance the controlled delivery of enzymes to patients with neurological and behavioral diseases and conditions susceptible to treatment with digestive enzymes.

In one aspect of the present disclosure the mucosal protectant sucralfate (tradename Carafate) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure the mucosal protectant bismuth subsalicylate (tradename Pepto-Bismol) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure coated or uncoated compositions comprising one or more digestive enzymes are utilized in combination with Pro-kinetic Agents to enhance the controlled delivery of enzymes to patients with neurological and behavioral diseases and conditions susceptible to treatment with digestive enzymes.

In one aspect of the present disclosure the pro-kinetic agent metoclopramide (tradename Reglan) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure the pro-kinetic agent bethanecol (tradename Urecholine) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure coated or uncoated compositions comprising one or more digestive enzymes are utilized in combination with Anticholinergic Agents to enhance the controlled delivery of enzymes to patients with neurological and behavioral diseases and conditions susceptible to treatment with digestive enzymes.

In one aspect of the present disclosure the anticholinergic agent scopolamine (tradename TransdermScop) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure the anticholinergic agent trihexyphenidyl (tradename Artane) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure the anticholinergic agent benztropine (tradename Cogentin) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure the anticholinergic agent dicyclomine (tradename Bentyl) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure the anticholinergic agent glycopyrrolate (tradename Robinul) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure the anticholinergic agent hyoscyamine (tradename Levsin) is utilized in conjunction with coated or uncoated compositions comprising one or more digestive enzymes to enhance the controlled delivery of enzymes to patients with neurological and behavioral diseases and conditions susceptible to treatment with digestive enzymes.

In one aspect of the present disclosure the anticholinergic agent atropine is utilized in conjunction with coated or uncoated compositions comprising one or more digestive enzymes to enhance the controlled delivery of enzymes to patients with neurological and behavioral diseases and conditions susceptible to treatment with digestive enzymes.

### DETAILED DESCRIPTION

Therapies to limit the ability of stomach acid to digest substrate do so either by limiting the amount of stomach acid or by limiting its contact with substrate. When digestive enzymes are administered orally, the enzymes are exposed to highly acidic conditions in the stomach, with a pH of 1or 2, as well as gastric proteases which denature and degrade the enzymes.

Gastric acid is secretion produced in the stomach. It is one of the main ditotonic solutions secreted, together with several enzymes and intrinsic factors. Chemically it is an acid solution with a pH of 1 to 2 in the stomach lumen, consisting mainly of hydrochloric acid (HCl) (around 0.5%, or 5000 parts per million), and large quantities of potassium chloride (KCl) and sodium chloride (NaCl).

Gastric acid is produced by parietal cells (also called oxyntic cells) in the stomach. Its secretion is a complex and relatively energetically expensive process. Parietal cells contain an extensive secretory network (called canaliculi) from which the gastric acid is secreted into the lumen of the stomach. These cells are part of epithelial fundic glands in the gastric mucosa. The pH of gastric acid is 2 to 3 in the human stomach lumen, the acidity being maintained by the proton pump H+/K+ ATPase (also referred to as the hydrogen ion pump herein). The parietal cell releases bicarbonate into the blood stream in the process, which causes the temporary rise of pH in the blood, known as alkaline tide.

The resulting highly acidic environment in the stomach lumen causes proteins from food to lose their characteristic folded structure (or denature). This exposes the protein's peptide bonds. The chief cells of the stomach secrete enzymes for protein breakdown (inactive pepsinogen and renin). Gastric acid activates pepsinogen into pepsin-this enzyme then helps digestion by breaking the bonds linking amino acids, a process known as proteolysis.

Gastric acid secretion happens in several steps. Chloride and hydrogen ions are secreted separately from the cytoplasm of parietal cells and mixed in the canaliculi. Gastric acid is then secreted into the lumen of the oxyntic gland and gradually reaches the main stomach lumen.

Chloride and sodium ions are secreted actively from the cytoplasm of the parietal cell into the lumen of the canaliculus. This creates a negative potential of -40 mV to -70 mV across the parietal cell membrane that causes potassium ions and a small number of sodium ions to diffuse from the cytoplasm into the parietal cell canaliculi.

The enzyme carbonic anhydrase catalyses the reaction between carbon dioxide and water to form carbonic acid. This acid immediately dissociates into hydrogen and bicarbonate ions. The hydrogen ions leave the cell through H+/K+ ATPase antiporter pumps. At the same time sodium ions are actively reabsorbed. This means the majority of secreted K+ and Na+ ions return to the cytoplasm. In the canaliculus, secreted hydrogen and chloride ions mix and are secreted into the lumen of the oxyntic gland.

The highest concentration that gastric acid reaches in the stomach is 160 mM in the canaliculi. This is about 3 million times that of arterial blood, but almost exactly isotonic with other bodily fluids. The lowest pH of the secreted acid is 0.8, but the acid is diluted in the stomach lumen to a pH between 1 and 3.

There are three phases in the secretion of gastric acid: 1. the cephalic phase: 30% of the total gastric acid to be produced is stimulated by anticipation of eating and the smell or taste of food; 2.the gastric phase: 60% of the acid secreted is stimulated by the distention of the stomach with food and digestion produces proteins, which causes even more gastrin production; and 3.the intestinal phase: the remaining 10% of acid is secreted when chyme enters the small intestine, and is stimulated by small intestine distention.

Gastric acid production is regulated by both the autonomic nervous system and several hormones. The parasympathetic nervous system, via the vagus nerve, and the hormone gastrin stimulate the parietal cell to produce gastric acid, both directly acting on parietal cells and indirectly, through the stimulation of the secretion of the hormone histamine from enterochromaffin-like cells (ECL). Vasoactive intestinal peptide, cholecystokinin, and secretin all inhibit production.

The production of gastric acid in the stomach is tightly regulated by positive regulators and negative feedback mechanisms. Four types of cells are involved in this process: parietal cells, G cells, D cells and enterochromaffine-like cells. Besides this, the endings of the vagus nerve (X) and the intramural nervous plexus in the digestive tract influence the secretion significantly.

Nerve endings in the stomach secrete two stimulatory neurotransmitters: acetylcholine and gastrin-releasing peptide. Their action is both direct on parietal cells and mediated through the secretion of gastrin from G cells and histamine from enterochromaffine-like cells. Gastrin acts on parietal cells directly and indirectly too, by stimulating the release of histamine.

The release of histamine is the most important positive regulation mechanism of the secretion of gastric acid in the stomach. Its release is stimulated by gastrin and acetylcholine and inhibited by somatostatin.

Parietal cells in the stomach produce acid necessary for digestion. Histamine release stimulates these cells to do so. Specific receptors on the parietal cell membrane, designated histamine-2 receptors, react to the presence of histamine at their active sites by beginning production of acid. Hydrogen ions, the essential building blocks of stomach acid, are then pumped into the stomach. Histamine-2 receptor antagonists (H2-blockers) are a class of drugs used to decrease the amount of acid produced in the stomach by inhibiting the ability of histamine to stimulate the histamine receptor on parietal cells. The acid-suppressing effects of most H2-blockers range from 6 to 24 hours. Examples of H2-blockers include Zantac®) (ranitidine), Axid® (nizatidine), Pepcid®) (famotidine) and Tagamet® (cimetidine).

Histamine 2-receptor blockers (H2-RBs) also come in various dosage forms. All come in a tablet form and ranitidine also comes in a soluble tablet as well as a syrup. Famotidine is available in a chewable tablet and a suspension.

Proton pump inhibitors (PPIs) are a class of drugs used to decrease the amount of acid produced in the stomach by irreversibly inhibiting the hydrogen ion pump from working. The acid-suppressing effects of most PPIs can last as long as 24 hours. Examples of PPIs include Prilosec® (omeprazole), Nexium® (esomeprazole), Zegerid® (omeprazole + sodium bicarbonate), Prevacid® (lansoprazole), Dexilant® (dexlansoprazole), AcipHex® (rabeprazole), and Protonix® (pantoprazole).

Proton-pump inhibitors (PPIs) come in various dosage forms. Examples of PPIs that come in a tablet form are pantoprazole, rabeprazole and omeprazole. Omeprazole, esomeprazole, lansoprazole and dexlansoprazole all come in capsule form and can either be swallowed whole or opened and their contents sprinkled over select foods or in select juices as noted by their respective manufacturers. Lansoprazole is also available as a soluble tablet. Esomeprazole is available in a granule formulation. Omeprazole also comes in a combination with NaHCO in capsule and granule formulations.

Mucosal Protectants provide a barrier that stomach acid must first penetrate before contacting digestive material and include Sucralfate, Bismuth Subsalicylate, Ranitidine Bismuth Citrate (RBC) and bismuth subgallate.

Sucralfate is a mucosal protectant that attenuates the power of stomach acid in digesting substrate by coating the GI tract and providing a barrier that acid must first penetrate before contacting digestible material. Sucralfate (Carafate) is a complex metal salt of sulfated sucrose. Although the sucralfate molecule contains aluminum hydroxide, the agent has little acid-neutralizing capacity. It is an aluminum salt of sulfated sucrose that disassociates under the acidic conditions in the stomach. It is speculated that the sucrose polymerizes and binds to protein in the ulcer crater to produce a kind of protective coating that can last for up to 6 hours. When exposed to gastric acid, the aluminum hydroxide dissociates, leaving sulfate anions that can bind electrostatically where it appears to form a protective barrier that may prevent further acid-peptic attack. Because of the lack of systemic absorption, sucralfate appears to have no systemic toxicity.

Similar agents include Pepto-Bismol®) (bismuth subsalicylate), ranitidine bismuth citrate (RBC) and bismuth subgallate. Bismuth preparations have been used widely to treat diarrhea, abdominal pain, and dyspepsia for hundreds of years. Two colloidal preparations of bismuth have been most commonly used, colloidal bismuth subcitrate and bismuth subsalicylate (e.g., Pepto-Bismol). The bismuth forms complexes with mucus to form a protective barrier that may prevent further acid-peptic attack. Bismuth is largely unabsorbed and is excreted in the feces.

Pro-kinetic Agents are typically used to increase lower esophageal sphincter (LES) pressure and also accelerate gastric emptying by stimulating the smooth muscles of the GI tract. Examples include Propulsid® (cisapride), Reglan® (metoclopramide), and Urecholine® (bethanecol).

The autonomic nervous system controls the body's involuntary activities. It is divided into two subsystems, the sympathetic and parasympathetic nervous systems. The sympathetic branch of the autonomic nervous system controls the body's involuntary "fight or flight" response. The parasympathetic branch is closely associated with the maintenance of function and homeostasis. It controls such things as respiration, genitourinary function and digestion. Parasympathetic nerves release a neurotransmitter called acetylcholine (ACh) to cause the contraction of muscles. Anticholinergic drugs inhibit parasympathetic nerve impulses by selectively blocking the binding of ACh. Anticholinergics therefore reduce colonic spasticity and decrease gastrointestinal secretions.

The median residence time of food in the stomachs of children was found by Fallingborg et al. (1990) to be 1.1 hours. The pH of the stomach was found to be below 3.

The small intestine is composed of the duodenum, jejunum, and ileum, extending from the stomach. The small intestine performs the majority of digestion and absorption of nutrients. Median small intestinal transit time in children was found by Fallingborg et al. (1990) to be 7.5 hours.

The duodenum is the section that curves around the head of the pancreas. The duodenum serves a mixing function as it combines digestive secretions from the pancreas with the contents expelled from the stomach. Enzymes from the pancreas that enter the duodenum further break down partly digested food from the stomach. The bulk of the digestion of proteins takes place in the duodenum before the material travels further into the small intestine. The pH of the duodenum rests at about 6.4.

The jejunum is the middle portion of the small intestine and passes imperceptibly into the ileum, the final portion of the small intestine, before entering the large intestine. The pH rises from the 6.4 of the duodenum to about 7.4 once reaching the ileum. Watson et al. (1972) observed a rise in pH values along the jejunum and ileum with respect to the duodenum as well.

The large intestine extends from the ileum of the small intestine to the anus. Median colonic transit time in children was found by Fallingborg et al. (1990) to be 17.5 hours.

Experiments carried out in animals, when it is possible to study absorption from small intestinal segments, indicate in general that there is a steady increase in amino acid absorption rates along the small intestine which is followed by a dramatic decline in the region of the terminal ileum (Lin and Wilson, 1960;Matthews and Laster, 1965; Schedl, Miller, Wilson, and Flores, 1969)

The combination of gastrointestinal modulator agents with compositions comprising one or more digestive enzymes in appropriate therapeutic dosages would be beneficial for enhancing their efficacy. Concerning agents that decrease the amount of acid present to digest substrate, more unaffected compositions comprising one or more digestive enzymes (coated or otherwise) would be able to make it to its target site in the small intestine. Concerning agents that limit the amount of time acid contacts substrate, compositions comprising one or more digestive enzymes (coated or otherwise) would arrive at its target site in the small intestine sooner and with less degradation.

Different dosage forms have different benefits. Tablets and capsules are the most common dosage forms for oral administration due to ease of manufacture, packaging and administration. Different forms of tablets have been primarily devised to meet the needs of select populations while maintaining the integrity of the active pharmaceutical ingredient. Some populations, notably infants and young children, cannot swallow tablets or capsules or find it difficult to do so. In these instances, a tablet that dissolves under the tongue, in the mouth, or in a specified liquid, or one that could be harmlessly chewed would be beneficial. Capsules that could be opened and their contents sprinkled over a small amount of food or in a liquid would also be beneficial. Any improvement that eases the administration of a necessary medication or lessens the antagonism associated with said administration, without compromising the effectiveness of the active pharmaceutical ingredient, is worthwhile.

Other types of solid dosage forms such as thinstrips, lollipops or gum bring a novel concept to the administration of medications to children. Aside from the obvious ease of administration from the viewpoint of the caregiver, there may be an added benefit. The administration of medication is oftentimes a private issue and the ability of a caregiver to provide a dose of medication in a seemingly matter-of-fact form may preserve that perception and instill in the user a mindset that views the administration as pleasant rather than monotonous or negative.

Liquid dosage forms also provide benefits of administration to infants and young children or anyone with compromised swallowing capability. Syrups, solutions and suspensions are easily swallowed. Unpleasant tastes can be masked by flavoring. An oral spray allows for the quick administration of a pre-measured dose of medication and supplies multiple metered doses in one handy device. With no need to aid swallowing (such as a glass of water, etc.) and the convenience of not having to rifle through a bottle of tablets, administration is simplified.

A tablet is a mixture of active substances and excipients, usually in powder form, pressed or compacted into a solid. The excipients include binders, glidants (flow aids) and lubricants to ensure efficient tableting; disintegrants to ensure that the tablet breaks up in the digestive tract; sweeteners or flavors to mask the taste of bad-tasting active ingredients; and pigments to make uncoated tablets visually attractive. A coating (sugar, enteric or film) may be applied to hide the taste of the tablet's components, to make the tablet smoother and easier to swallow, and to make it more resistant to the environment, extending its shelf life. Tablets may be buffered (by potassium metaphosphate, potassium phosphate, monobasic sodium acetate, etc.) to combat change in pH. Tablets may be delayed-release, sustained-release, extended-release, controlled- delivery, long-acting, orally-disintegrating or melts, among others, often denoting the pharmacokinetic profile of the active agent. A capsule-shaped tablet is a caplet.

Some tablets may be taken sublingually or allowed to dissolve in the mouth. The principle behind sublingual administration is simple. When a chemical comes in contact with the mucous membrane beneath the tongue, or buccal mucosa, it diffuses through it. Because the connective tissue beneath the epithelium contains a profusion of capillaries, the substance then diffuses into them and enters the venous circulation. Troches are medicated lozenges designed to dissolve in the mouth. Soluble tablets dissolve on contact with the tongue.

Slurry may be made when a dissolvable tablet containing a gelling agent is added to a liquid.

Tablets may also be micro-coated and placed in a capsule for administration.

In the manufacture of pharmaceuticals, encapsulation refers to a range of techniques used to enclose medicines in a relatively stable shell known as a capsule, allowing them to, for example, be taken orally or be used as suppositories. The two main types of capsules are hard-shelled capsules, which are normally used for dry, powdered ingredients, and soft-shelled capsules, primarily used for oils and for active ingredients that are dissolved or suspended in oil. Both of these classes of capsule are made both from gelatin and from plant-based gelling substances like carrageenans and modified forms of starch and cellulose, and the latter form is usually seemless. Capsules are made in two parts by dipping metal rods in molten gelatin solution. The capsules are supplied as closed units to the pharmaceutical manufacturer. Before use, the two halves are separated, the capsule is filled with powder (either by placing a compressed slug of powder into one half of the capsule, or by filling one half of the capsule with loose powder) and the other half of the capsule is pressed on. The advantage of inserting a slug of compressed powder is that control of weight variation is better, but the machinery involved is more complex.

Sprinkle capsules are a dosage form consisting of small beads or granules of an active drug contained in a capsule that can be readily administered by simply opening up the capsule and distributing the contents over something to be swallowed.

A suspension is a heterogeneous fluid containing solid particles that are sufficiently large for sedimentation. Usually they must be larger than 1 micrometer. The internal phase (solid) is dispersed throughout the external phase (fluid) through mechanical agitation, with the use of certain excipients or suspending agents. Unlike colloids, suspensions will eventually settle. An example of a suspension would be sand in water. The suspended particles are visible under a microscope and will settle over time if left undisturbed. This distinguishes a suspension from a colloid in which the suspended particles are smaller and do not settle. Colloids and suspensions are different from a solution, in which the dissolved substance (solute) does not exist as a solid and solvent and solute are homogeneously mixed. Oftentimes, powders of active ingredients may be packaged such that the addition of a diluent dissolves the powder and holds it in a liquid suspension.

When used as a pharmaceutical preparation, elixirs contain an active ingredient that is dissolved in a solution that contains some percentage (usually 40-60%) of ethyl alcohol and is designed to be taken orally.

Syrups are oftentimes employed as a base for medicinal purposes and consist of a concentrated or saturated solution of refined sugar in distilled water.

A suspension of liquid droplets or fine solid particles in a gas is called an aerosol. This can take the form of an oral spray.

A gum may be devised whereby an active ingredient is incorporated into a vegetative resinous substance (e.g. acacia) and released via the actual mechanical effect of chewing or the action of saliva on the gum itself.

A thinstrip is an active pharmaceutical product coated by a lipid layer designed to dissolve in the mouth over a brief period of time. The same technology could be used to produce a medicated lollipop for transmucosal delivery.

In pharmaceutical terms, a granule is a small particle gathered into a larger, permanent aggregate in which the original particles can still be identified.

Representative dosages for each of the combination formulations is presented below by drug, disease, and appropriate age category. In addition, dosages are provided for those who are renally or hepatically impaired.

It should be noted that the use of these combinations in pregnancy may have risks as identified by the FDA Pregnancy Category Guidelines. Specifically, omeprazole and omeprazole/sodium bicarbonate, as well as bismuth subsalicylate are designated as Pregnancy Category C. All others listed below are designated Pregnancy Category B.

In some embodiments, digestive enzyme compositions may be combined with gastrointestinal modulators of stomach acid for use in treating individuals with neurological, behavioral, infectious, or genetic diseases or conditions susceptible to treatment as specified herein.

In some embodiments, a digestive enzyme composition may be coated; such coated compositions may be referred to as enzyme preparations herein. In some embodiments, the digestive enzymes can be lipid-coated. In some embodiments, the one or more digestive enzymes comprise one or more enzymes selected from the group consisting of proteases, amylases, celluloses, sucrases, maltases, papain (e.g., from papaya), bromelain (e.g., from pineapple), hydrolases, and lipases. In some embodiments, the one or more digestive enzymes comprise one or more pancreatic enzymes. In some embodiments, the pharmaceutical composition comprises one or more proteases, one or more lipases, and one or more amylases. In some embodiments, the one or more proteases comprise chymotrypsin and trypsin.

The one or more digestive enzymes are, independently, derived from an animal source, a microbial source, a fungal source, or a plant source, or are synthetically prepared. In some embodiments, the animal source is a pig, e.g., a pig pancreas, or avian, e.g., "bird" proventriculus or small intestine.

In some embodiments, the digestive enzyme composition is comprised of protease, lipase, and amylase where the activities are: protease between 10,000 to 1,500,000 U.S.P. units including 10,000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 1,050,000, 1,100,000, 1,150,000, 1,200,00, 1,250,000, 1,300,000, 1,350,000, 1,400,000, 1,450,000, and 1,500,000 along with all values in-between per dose and where the ratio of protease to lipase is such that the amount of lipase is never more than 0.188 times the amount of protease and where the ratio of protease activity to amylase activity is between 1:0.1 and 1:10.

In some embodiments, the digestive enzyme composition is comprised of protease and lipase, where the activities are: protease between 10,000 to 1,500,000 U.S.P, units including 10,000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 1,050,000, 1,100,000, 1,150,000, 1,200,00, 1,250,000, 1,300,000, 1,350,000, 1,400,000, 1,450,000, and 1,500,000 along with all values in-between per dose and where the ratio of protease to lipase is such that the amount of lipase is never more than 0.188 times the amount of protease.

In some embodiments, the digestive enzyme composition is comprised of protease and amylase where the activities are: protease between 10,000 to 1,500,000 U.S.P, units including 10,000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 1,050,000, 1,100,000, 1,150,000, 1,200,00, 1,250,000, 1,300,000, 1,350,000, 1,400,000, 1,450,000, and 1,500,000 along with all values in-between per dose and where the ratio of protease activity to amylase activity is between 1:0.1 and 1:10.

In some embodiments, the digestive enzyme composition is comprised of protease where the activities are: protease between 10,000 to 1,500,000 U.S.P, units including 10,000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 1,050,000, 1,100,000, 1,150,000, 1,200,00, 1,250,000, 1,300,000, 1,350,000, 1,400,000, 1,450,000, and 1,500,000 along with all values in-between per dose.

In some embodiments, the digestive enzyme composition is comprised of protease, lipase, and amylase where the activities are: protease between 10,000 to 1,500,000 U.S.P, units including 10,000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 1,050,000, 1,100,000, 1,150,000, 1,200,00, 1,250,000, 1,300,000, 1,350,000, 1,400,000, 1,450,000, and 1,500,000 along with all values in-between per dose; lipases from about 1,880 to about 282,000 U.S.P. units including, 1,880, 10,000, 50,000, 100,000, 150,000, 200,000, 250,000, 282,000, along with all values in-between per dose; amylases from about 1,000 to about 15,000,000 U.S.P. units, including 1,000, 10,000, 100,000, 500,000, 1,000,000, 2,000,000, 3,000,000, 4,000,000, 5,000,000, 6,000,000, 7,000,000, 8,000,000, 9,000,000, 10,000,000, 11,000,000, 12,000,000, 13,000,000, 14,000,000, 15,000,000, U.S.P. units, along with all values in-between per dose.

In some embodiments, the digestive enzyme composition is comprised of protease and lipase, where the activities are: protease between 10,000 to 1,500,000 U.S.P, units including 10,000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 1,050,000, 1,100,000, 1,150,000, 1,200,00, 1,250,000, 1,300,000, 1,350,000, 1,400,000, 1,450,000, and 1,500,000 along with all values in-between per dose; lipases from about 1,880 to about 282,000 U.S.P. units including, 1,880, 10,000, 50,000, 100,000, 150,000, 200,000, 250,000, 282,000, along with all values in-between per dose.

In some embodiments, the digestive enzyme composition is comprised of protease and amylase where the activities are: protease between 10,000 to 1,500,000 U.S.P, units including 10,000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 1,050,000, 1,100,000, 1,150,000, 1,200,00, 1,250,000, 1,300,000, 1,350,000, 1,400,000, 1,450,000, and 1,500,000 along with all values in-between per dose; amylases from about 1,000 to about 15,000,000 U.S.P. units, including 1,000, 10,000, 100,000, 500,000, 1,000,000, 2,000,000, 3,000,000, 4,000,000, 5,000,000, 6,000,000, 7,000,000, 8,000,000, 9,000,000, 10,000,000, 11,000,000, 12,000,000, 13,000,000, 14,000,000, 15,000,000, U.S.P. units, along with all values in-between per dose.

In some embodiments, the digestive enzyme composition is comprised of protease, where the activities are: protease between 10,000 to 1,500,000 U.S.P, units including 10,000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 1,050,000, 1,100,000, 1,150,000, 1,200,00, 1,250,000, 1,300,000, 1,350,000, 1,400,000, 1,450,000, and 1,500,000 along with all values in-between per dose.

In some embodiments, the digestive enzyme composition comprises at least one amylase, a mixture of proteases comprising chymotrypsin and trypsin, and at least one lipase. The pharmaceutical composition can further include papain, e.g., from papaya. In some embodiments, the coated pharmaceutical composition comprises per dose: amylases from about 1,000 to about 15,000,000 U.S.P. units, including 1,000, 10,000, 100,000, 500,000, 1,000,000, 2,000,000, 3,000,000, 4,000,000, 5,000,000, 6,000,000, 7,000,000, 8,000,000,9,000,000, 10,000,000, 11,000,000, 12,000,000, 13,000,000, 14,000,000, 15,000,000, U.S.P. units, along with all values in-between; proteases from about 10,000 to about 1,500,000 U.S.P, units including 10,000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 1,050,000, 1,100,000, 1,150,000, 1,200,00, 1,250,000, 1,300,000, 1,350,000, 1,400,000, 1,450,000, and 1,500,000 along with all values in-between, lipases from about 1,880 to about 282,000 U.S.P. units including, 1,880, 10,000, 50,000, 100,000, 150,000, 200,000, 250,000, 282,000 , along with all values in-between.,.

In some embodiments, the digestive enzyme composition comprises at least one protease and at least one lipase, wherein the ratio of total proteases to total lipases (in USP units) ranges from about 5.371:1 to about 20:1 including 5.371:1, 6:1, 7:1, 8:1, 9:1, 10:1, 11; 1, 12; 1, 13;1, 14:1, 15:1, 16;1, 17:1, 18:1, 19:1 and 20:1, along with all values in-between. In some embodiments, the ratio of proteases to lipases ranges from about 5.371:1 to about 10:1 including 5.371:1, 6:1, 7:1, 8:1, 9:1, and 10:1, along with all values in-between.

In some embodiments a coated digestive enzyme preparation comprising (a) a core containing a digestive enzyme particle, where the enzyme present in an amount of from about 5% to 95% by weight of the particles, including 5%, 10%, 15%, 20%, 25%, 30%, 35%, 40%, 45%, 50%, 55%, 60%, 65%, 70%, 75%, 80%, 85%, 90% and 95% by weight, along with all values in-between; and (b) a coating comprising a crystallizable lipid, wherein the coating continuously coats the core and the crystallizable lipid releases the enzyme upon exposure to physiological conditions.

In some embodiments a coated enzyme preparation having particles which comprise: (a) a core comprising pancreatic or other digestive enzymes present in an amount of from about 5% to 95% by weight of the particles, including 5%, 10%, 15%, 20%, 25%, 30%, 35%, 40%, 45%, 50%, 55%, 60%, 65%, 70%, 75%, 80%, 85%, 90% and 95% by weight along with all values in-between; and (b) a generally uniform coating to provide for controlled release of the enzymes, the coating comprising a crystallizable lipid. In some embodiments, the coated enzyme preparation particles of the enzyme delivery system are non-aerosolizable.

In some embodiments a pharmaceutical dosage comprising a population of extended release beads, wherein said extended release beads comprise: an active-containing core particle comprising pancreatic or other digestive enzymes as the active; and an extended release coating comprising a water insoluble polymer membrane surrounding said core, wherein said water insoluble polymer membrane comprises a polymer selected from the group consisting of ethers of cellulose, esters of cellulose, cellulose acetate, ethyl cellulose, polyvinyl acetate, neutral copolymers based on ethyl acrylate and methyl methacrylate, copolymers of acrylic and methacrylic acid esters with quaternary ammonium groups, pH-insensitive ammonio methacrylic acid copolymers, and mixtures thereof; wherein the total amount of pancreatin in the pharmaceutical dosage form contains between 15,000 USP units protease to 1.5 million USP units protease per dose and where the ratio of protease to lipase is such that the amount of lipase is never more than 0.188 times the amount of protease and where the ratio of protease activity to amylase activity is between 1:0.1 and 1:10

In some embodiments the extended release coating further comprises a water soluble polymer selected from the group consisting of methylcellulose, hydroxypropylcellulose, hydroxypropyl methylcellulose, polyethylene glycol, polyvinylpyrrolidone and mixtures thereof.

In some embodiments the extended release coating further comprises a plasticizer selected from the group consisting of triacetin, tributyl citrate, triethyl citrate, acetyl tri-n-butyl citrate, diethyl phthalate, dibutyl sebacate, polyethylene glycol, polypropylene glycol, castor oil, acetylated mono- and di-glycerides, and mixtures thereof.

In some embodiments, the combination compositions according to this disclosure produce enzyme preparations, including coated enzyme preparations, characterized, for example, by controlled rates of release, reduction in aerosolization and safer administration, ability to be administered by a sprinkle/sachet delivery method, improved flow characteristics, enhanced shelf life and storage capacity, and other properties described herein. In other aspects, the coated enzyme preparation has improved pour properties which facilitate manufacturing and packaging processes, for example packaging in pouches and sachets.

Some coated digestive enzyme preparations which comprise a coating of a crystallizable lipid and a digestive enzyme core have favorable release and activity profiles and permit site time specific and/or location specific targeted release along the GI tract. In some aspects, the coated digestive enzyme preparations are prepared to obtain specific delivery times or specific regions within the human gastrointestinal (GI) tract. In some embodiments, the crystallizable lipid composition is hydrogenated soybean oil, but may be any suitable crystallizable lipid or lipid blend. Additionally the coating of the coated digestive enzyme preparations may be tailored for optimal targeted release of the enzyme(s) to achieve maximal combined efficacy of the enzymes when used in conjunction with acid reducer(s).

Some embodiments utilize stable enzyme preparations protected against the environment to reduce, for example, degradation and/or denaturation of the enzymes. This permits delivery of more accurate doses of the enzyme preparation to treated individuals. The coating can also, in some aspects, provide emulsification when the enzyme preparations are contacted with appropriate solvents, while also surprisingly providing for controlled release of the enzyme in the gastrointestinal (GI) system. The emulsification properties of the coating in a solvent allows for controlled release of the enzyme, preferably at selected locations in the GI tract, where enzyme utilization provides the most effective prophylaxis or treatment.

In another embodiment enzyme preparations are utilized with lipid coating of enzymes. The method of making the preparations comprises providing a crystallizable lipid, and coating size-specific pancreatic or other digestive enzyme particles as described herein with the lipid. The digestive enzymes comprise 5% to 95% by weight of the coated enzyme preparations, including 5%, 10%, 15%, 20%, 25%, 30%, 35%, 40%, 45%, 50%, 55%, 60%, 65%, 70%, 75%, 80%, 85%, 90% and 95%, along with all values in-between.

The methods of this disclosure can be used to produce coated digestive enzyme preparations comprising digestive enzymes coated with a crystallizable lipid alone, or with a lipid blend to achieve a controlled rate of enzyme release, with increased release of the digestive enzyme upon exposure of the coated preparation to a suitable solvent. Coated digestive enzyme preparations having a coating comprising one or more monoglycerides exhibit increased release of the digestive enzyme upon exposure of the coated composite to a solvent, such as water, while protecting against release in 0.1 N HCl.

In one example of the present disclosure the release of digestive enzymes is timed to release specific percentages of enzymes in specific portions of the gastrointestinal tract by a combined use of gastrointestinal modulators and coating technologies.

In another example of the present disclosure the amount and or types of gastrointestinal modulators are varied around a fixed enzyme coating to maximize efficacious release of digestive enzymes in the appropriate portion(s) of the gastrointestinal tract to treat the symptoms or causes of one or more diseases.

In another example of the present disclosure the amount and or types of gastrointestinal modulators are fixed and the thickness of the enzyme coating is varied to maximize efficacious release of digestive enzymes in the appropriate portion(s) of the gastrointestinal tract to treat the symptoms or causes of one or more diseases.

In another example of the present disclosure the timing of giving one or more gastrointestinal modulators are varied and the thickness of the coating is fixed to maximize efficacious release of digestive enzymes in the appropriate portion(s) of the gastrointestinal tract to treat the symptoms or causes of one or more diseases.

In another example of the present disclosure the coatings and enzymes are concentrically nested to allow timed release of enzymes in more than one portion of the gastrointestinal tract to treat the symptoms or causes of one or more diseases.

One example of a formulation of a therapeutically effective amount of coated or uncoated digestive enzymes in combination with ranitidine for the treatment of Autism, ADD, ADHD and other pervasive developmental disorders in patients ranging in age from 3 to 16 years is 0.2 to 10 mg/kg, in up to 2 divided doses, up to a maximum daily dose of 300 mg/day in tablet, EFFERdose® tablet, capsule, EFFERdose® granule or syrup. A typical dose of ranitidine might be 75 mg given 30 minutes prior to the first digestive enzyme administration of the day. A typical dosing of coated or uncoated digestive enzymes in combination with ranitidine is 4,300 U.S.P. units of protease per kilogram three times per day in patients ranging in age from 3 to 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated composition comprising one or more digestive enzymes in combination with ranitidine for the treatment of Familial Dysautonomia in patients ranging in age from 3 to 16 years is 0.2 to 10 mg/kg, in up to 2 divided doses, up to a maximum daily dose of 300 mg/day in tablet, EFFERdose® tablet, capsule, EFFERdose®) granule or syrup. A typical dose of ranitidine might be 75 mg given 30 minutes prior to the first composition comprising one or more digestive enzymes administration of the day. A typical dosing of coated or uncoated digestive enzymes in combination with ranitidine is 4,300 U.S.P. units of protease per kilogram three times per day in patients ranging in age from 3 to 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated composition comprising one or more digestive enzymes in combination with ranitidine for the treatment of Guillain-Barre Syndrome in patients ranging in age from 3 to 16 years is 0.2 to 10 mg/kg, in up to 2 divided doses, up to a maximum daily dose of 300 mg/day in tablet, EFFERdose® tablet, capsule, EFFERdose® granule or syrup. A typical dose of ranitidine might be 75 mg given 30 minutes prior to the first enzyme composition administration of the day. A typical dosing of coated or uncoated digestive enzymes in combination with ranitidine is 4,300 U.S.P. units of protease per kilogram three times per day in patients ranging in age from 3 to 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated composition comprising one or more digestive enzymes in combination with ranitidine for the treatment of neuroblastoma in patients ranging in age from 3 to 16 years is 0.2 to 10 mg/kg, in up to 2 divided doses, up to a maximum daily dose of 300 mg/day in tablet, EFFERdose® tablet, capsule, EFFERdose®) granule or syrup. A typical dose of ranitidine might be 75 mg given 30 minutes prior to the first digestive enzyme administration of the day. A typical dosing of coated or uncoated digestive enzymes in combination with ranitidine is 4,300 U.S.P. units of protease per kilogram three times per day in patients ranging in age from 3 to 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in patients ranging in age from 3 to 16 years is 0.2 to 10 mg/kg, in up to 2 divided doses, up to a maximum daily dose of 300 mg/day in tablet, EFFERdose® tablet, capsule, EFFERdose® granule or syrup. A typical dose of ranitidine might be 75 mg given 30 minutes prior to the first PEC administration of the day. A typical dosing of coated or uncoated digestive enzymes in combination with ranitidine is 4,300 U.S.P. units of protease per kilogram three times per day in patients ranging in age from 3 to 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of diabetic cardiovascular neuropathy in patients ranging in age from 3 to 16 years is 0.2 to 10 mg/kg, in up to 2 divided doses, up to a maximum daily dose of 300 mg/day in tablet, EFFERdose® tablet, capsule, EFFERdose® granule or syrup. A typical dose of ranitidine might be 75 mg given 30 minutes prior to the first PEC administration of the day. A typical dosing of coated or uncoated digestive enzymes in combination with ranitidine is 4,300 USP per kilogram three times per day in patients ranging in age from 3 to 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Complex Regional Pain Syndrome in patients ranging in age from 3 to 16 years is 0.2 to 10 mg/kg, in up to 2 divided doses, up to a maximum daily dose of 300 mg/day in tablet, EFFERdose® tablet, capsule, EFFERdose® granule or syrup. A typical dose of ranitidine might be 75 mg given 30 minutes prior to the first PEC administration of the day. A typical dosing of coated or uncoated digestive enzymes in combination with ranitidine is 4,300 U.S.P. units of protease per kilogram three times per day in patients ranging in age from 3 to 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Bipolar Disorder, Obsessive Compulsive Disorder or Oppositional Defiant Disorder in patients ranging in age from 3 to 16 years is 0.2 to 10 mg/kg, in up to 2 divided doses, up to a maximum daily dose of 300 mg/day in tablet, EFFERdose®) tablet, capsule, EFFERdose®) granule or syrup. A typical dose of ranitidine might be 75 mg given 30 minutes prior to the first PEC administration of the day. A typical dosing of coated or uncoated digestive enzymes in combination with ranitidine is 4,300 U.S.P. units of protease per kilogram three times per day in patients ranging in age from 3 to 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of the symptoms of addiction in patients ranging in age from 3 to 16 years is 0.2 to 10 mg/kg, in up to 2 divided doses, up to a maximum daily dose of 300 mg/day in tablet, EFFERdose®) tablet, capsule, EFFERdose®) granule or syrup. A typical dose of ranitidine might be 75 mg given 30 minutes prior to the first PEC administration of the day. A typical dosing of coated or uncoated digestive enzymes in combination with ranitidine is 4,300 U.S.P. units of protease per kilogram three times per day in patients ranging in age from 3 to 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of William's Syndrome in patients ranging in age from 3 to 16 years is 0.2 to 10 mg/kg, in up to 2 divided doses, up to a maximum daily dose of 300 mg/day in tablet, EFFERdose® tablet, capsule, EFFERdose® granule or syrup. A typical dose of ranitidine might be 75 mg given 30 minutes prior to the first PEC administration of the day. A typical dosing of coated or uncoated digestive enzymes in combination with ranitidine is 4,300 U.S.P. units of protease per kilogram three times per day in patients ranging in age from 3 to 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Cystic Fibrosis in patients ranging in age from 3 to 16 years is 0.2 to 10 mg/kg, in up to 2 divided doses, up to a maximum daily dose of 300 mg/day in tablet, EFFERdose® tablet, capsule, EFFERdose® granule or syrup. A typical dose of ranitidine might be 75 mg given 30 minutes prior to the first PEC administration of the day. A typical starting dosing of coated or uncoated digestive enzymes in combination with ranitidine for cystic fibrosis is 2,500 U.S.P. units of lipase per kilogram three times per day in patients ranging in age from 4 years and older. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Prion Diseases in patients ranging in age from 3 to 16 years is 0.2 to 10 mg/kg, in up to 2 divided doses, up to a maximum daily dose of 300 mg/day in tablet, EFFERdose® tablet, capsule, EFFERdose® granule or syrup. A typical dose of ranitidine might be 75 mg given 30 minutes prior to the first PEC administration of the day. A typical dosing of coated or uncoated digestive enzymes in combination with ranitidine is 4,300 U.S.P. units of protease per kilogram three times per day in patients ranging in age from 3 to 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Autism, ADD, ADHD and other pervasive developmental disorders in adult patients is 15-150 mg once or twice daily, up to a maximum daily dose of 300 mg/day, or 300 mg once daily in tablet, EFFERdose® tablet, capsule, EFFERdose® granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Parkinson's in adult patients is 15-150 mg once or twice daily, up to a maximum daily dose of 300 mg/day, or 300 mg once daily in tablet, EFFERdose® tablet, capsule, EFFERdose® granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Familial Dysautonomia in adult patients is 15-150 mg once or twice daily, up to a maximum daily dose of 300 mg/day, or 300 mg once daily in tablet, EFFERdose®) tablet, capsule, EFFERdose®) granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Guillain-Barre Syndrome in adult patients is 15-150 mg once or twice daily, up to a maximum daily dose of 300 mg/day, or 300 mg once daily in tablet, EFFERdose®) tablet, capsule, EFFERdose®) granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of neuroblastoma in adult patients is 15-150 mg once or twice daily, up to a maximum daily dose of 300 mg/day, or 300 mg once daily in tablet, EFFERdose® tablet, capsule, EFFERdose® granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adult patients is 15-150 mg once or twice daily, up to a maximum daily dose of 300 mg/day, or 300 mg once daily in tablet, EFFERdose®) tablet, capsule, EFFERdose®) granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of diabetic cardiovascular neuropathy in adult patients is 15-150 mg once or twice daily, up to a maximum daily dose of 300 mg/day, or 300 mg once daily in tablet, EFFERdose® tablet, capsule, EFFERdose® granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Complex Regional Pain Syndrome in adult patients is 15-150 mg once or twice daily, up to a maximum daily dose of 300 mg/day, or 300 mg once daily in tablet, EFFERdose® tablet, capsule, EFFERdose® granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Bipolar Disorder, Obsessive Compulsive Disorder or Oppositional Defiant Disorder in adult patients is 15-150 mg once or twice daily, up to a maximum daily dose of 300 mg/day, or 300 mg once daily in tablet, EFFERdose® tablet, capsule, EFFERdose® granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of symptoms of addiction in adult patients is 15-150 mg once or twice daily, up to a maximum daily dose of 300 mg/day, or 300 mg once daily in tablet, EFFERdose® tablet, capsule, EFFERdose® granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of William's Syndrome in adult patients is 15-150 mg once or twice daily, up to a maximum daily dose of 300 mg/day, or 300 mg once daily in tablet, EFFERdose® tablet, capsule, EFFERdose® granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage..

One example of a formulation of coated or uncoated PEC in combination with ranitidine for the treatment of Cystic Fibrosis in adult patients is 15-150 mg once or twice daily, up to a maximum daily dose of 300 mg/day, or 300 mg once daily in tablet, EFFERdose® tablet, capsule, EFFERdose® granule or syrup. A typical dose of ranitidine might be 150 mg given 30 minutes prior to the first PEC administration of the day. A typical starting dosing of coated or uncoated digestive enzymes in combination with ranitidine for cystic fibrosis is 2,500 USP units of lipase per kilogram three times per day in patients ranging in age from 16 years and older. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Prion Diseases in adult patients is 15-150 mg once or twice daily, up to a maximum daily dose of 300 mg/day, or 300 mg once daily in tablet, EFFERdose® tablet, capsule, EFFERdose® granule or syrup. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day in patients over 16 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Autism, ADD, ADHD and other pervasive developmental disorders in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Parkinson's in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for Patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Familial Dysautonomia in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for Patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Guillain-Barre Syndrome in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of neuroblastoma in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of diabetic cardiovascular neuropathy in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Complex Regional Pain Syndrome in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Alzheimer's Disease in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Bipolar Disorder, Obsessive Compulsive Disorder or Oppositional Defiant Disorder in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of symptoms of addiction in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of William's Syndrome in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Cystic Fibrosis in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical starting dosing of coated or uncoated digestive enzymes in combination with ranitidine for cystic fibrosis is 2,500 U.S.P. units of lipase per kilogram three times per day in patients ranging in age from 4 years and older. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with ranitidine for the treatment of Prion Diseases in elderly or patients with impaired renal function (creatinine clearance <50 mL/min) is 15-150 mg every 24 hours. The frequency of dosing may be increased to every 12 hours with caution. The dosing schedule should also be adjusted to coincide with the end of hemodialysis as ranitidine is cleared by hemodialysis. A typical dose of ranitidine might be 150 mg given 30 minutes prior to the first PEC administration of the day. A typical dosing of coated or uncoated digestive enzymes in combination with ranitidine is 4,300 U.S.P. units of protease per kilogram three times per day for patients over 65 years of age. Dosing for both the enzyme composition or enzyme preparation and ranitidine may be from the minimal clinically proven safe and efficacious dosing to the maximal proven safe and efficacious dosage.

One example of a formulation of coated or uncoated PEC in combination with nizatidine for the treatment of Autism, ADD, ADHD and other pervasive developmental disorders in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Parkinson's in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Familial Dysautonomia in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Guillain-Barre Syndrome in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of neuroblastoma in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of diabetic cardiovascular neuropathy in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Complex Regional Pain Syndrome in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Alzheimer's Disease in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Bipolar Disorder, Obsessive Compulsive Disorder or Oppositional Defiant Disorder in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of symptoms of addiction in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of William's Syndrome in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Cystic Fibrosis in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 2500 U.S.P. units of lipase per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Prion Diseases in adult patients is 15-150 mg once or twice daily, up to a maximum daily dose of 300 mg/day, or 15-300 mg once daily in capsule form. A typical dose of nizatidine might be 150 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Autism, ADD, ADHD and other pervasive developmental disorders in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Parkinson's in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Familial Dysautonomia in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day in patients over 16 years of age. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Guillain-Barre Syndrome in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of neuroblastoma in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of diabetic cardiovascular neuropathy in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Complex Regional Pain Syndrome in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Alzheimer's Disease in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Bipolar Disorder, Obsessive Compulsive Disorder or Oppositional Defiant Disorder in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of symptoms of addiction in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of William's Syndrome in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Cystic Fibrosis in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical start dosing of coated or uncoated digestive enzymes in combination with nizatidine is 2,500 U.S.P. units of lipase per kilogram three times per day for children older than 4 years and Adults. A typical starting dose of coated or uncoated digestive enzymes in children older than 12 months but younger than 4 years in 1,000 Lipase USP per kilogram and in infants up to 12 months from 2,000 to 4,000 lipase units per 120mL of formula or per breast feeding. For Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with nizatidine for the treatment of Prion Diseases in patients with a creatinine clearance <20 mL/min is 15-150 mg every other day. In patients with a creatinine clearance of 20-50 mL/min the dose is 15-150 mg daily. A typical dose of nizatidine might be 75 mg given 30 minutes prior to the first PEC administration of the day. A typical dosing of coated or uncoated digestive enzymes in combination with nizatidine is 4,300 U.S.P. units of protease per kilogram three times per day. Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with famotidine for the treatment of Autism, ADD, ADHD and other pervasive developmental disorders in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 3 to 16 years is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Familial Dysautonomia in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 3 to 16 years is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Guillain-Barre Syndrome in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 3 to 16 years is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of neuroblastoma in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 3 to 16 years is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 3 to 16 years is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of diabetic cardiovascular neuropathy in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 3 to 16 years is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Complex Regional Pain Syndrome in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 3 to 16 years is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Bipolar Disorder, Obsessive Compulsive Disorder or Oppositional Defiant Disorder in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 3 to 16 years is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of symptoms of addiction in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 3 to 16 years is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of William's Syndrome in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 3 to 16 years is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Cystic Fibrosis in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 4 to 16 years is 2,500 U.S.P. units of lipase per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Prion Diseases in patients ranging in age from 3 to 16 years is 0.05-2 mg/kg/day divided twice daily up to a maximum daily dose of 80 mg/day in tablet, RPD tablet or suspension. A typical dose of famotidine might be 10 mg given 30 minutes before the first PEC administration of the day. A typical dosing of coated or uncoated digestive enzymes in combination with famotidine for children ages 3 to 16 years is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Autism, ADD, ADHD and other pervasive developmental disorders in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Parkinson's in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Familial Dysautonomia in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Guillain-Barre Syndrome in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of neuroblastoma in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adult patients is 1-80 mg/day in tablet, RPD tablet or suspension. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of diabetic cardiovascular neuropathy in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Complex Regional Pain Syndrome in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Alzheimer's Disease in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Bipolar Disorder, Obsessive Compulsive Disorder or Oppositional Defiant Disorder in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of symptoms of addiction in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of William's Syndrome in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Cystic Fibrosis in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 2,500 U.S.P. units of lipase per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Prion Diseases in adult patients is 1-80 mg/day in tablet, RPD tablet or suspension. A typical dose of famotidine might be 20 mg given 30 minutes before the first PEC administration of the day. A typical dosing of coated or uncoated digestive enzymes in combination with famotidine for ages 16 years and up is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Autism, ADD, ADHD and other pervasive developmental disorders in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Parkinson's in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Familial Dysautonomia in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Guillain-Barre Syndrome in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of neuroblastoma in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of diabetic cardiovascular neuropathy in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Complex Regional Pain Syndrome in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Alzheimer's Disease in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Bipolar Disorder, Obsessive Compulsive Disorder or Oppositional Defiant Disorder in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of symptoms of addiction in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of William's Syndrome in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with famotidine for the treatment of Cystic Fibrosis in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical start dosing of coated or uncoated digestive enzymes in combination with nizatidine is 2,500 U.S.P. units of lipase per kilogram three times per day for children older than 4 years and Adults. A typical starting dose of coated or uncoated digestive enzymes in children older than 12 months but younger than 4 years in 1,000 Lipase USP per kilogram and in infants up to 12 months fom 2,000 to 4,000 lipase units per 120mL of formula or per breast feeding. For Dosing for both the enzyme composition or enzyme preparation and nizatidine may be from the minimal clinically proven safe and efficacious dosing to the maximal proven safe and efficacious dosage.

One example of a formulation of coated or uncoated PEC in combination with famotidine for the treatment of Prion Diseases in patients with impaired renal function is 1-40 mg/day or 2-80 mg every 36 to 48 hours in tablet, RPD tablet or suspension. A typical dose of famotidine might be 20 mg given 30 minutes before the first PEC administration of the day. A typical dosing of coated or uncoated digestive enzymes in combination with famotidine is 4,300 U.S.P. units of protease per kilogram three times per day. Dosing for both the enzyme composition or enzyme preparation and famotidine may be from the minimal clinically proven safe and efficacious dosing to the maximal proven safe and efficacious dosage.

One example of a formulation of coated or uncoated PEC in combination with cimetidine for the treatment of Autism, ADD, ADHD and other pervasive developmental disorders in adult patients is 20-1600 mg at bedtime in tablet or liquid. A typical dose of cimetidine might be 200 mg given at bedtime. A typical dosing of coated or uncoated digestive enzymes in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Parkinson's in adult patients is 20-1600 mg at bedtime in tablet or liquid. A typical dose of cimetidine might be 200 mg given at bedtime. A typical dosing of coated or uncoated digestive enzymes for patients over the age of 16 in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Familial Dysautonomia in adult patients is 20-1600 mg at bedtime in tablet or liquid. A typical dose of cimetidine might be 200 mg given at bedtime. A typical dosing of coated or uncoated digestive enzymes for patients over the age of 16 in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Guillain-Barre Syndrome in adult patients is 20-1600 mg at bedtime in tablet or liquid. A typical dose of cimetidine might be 200 mg given at bedtime. A typical dosing of coated or uncoated digestive enzymes for patients over the age of 16 in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of neuroblastoma in adult patients is 20-1600 mg at bedtime in tablet or liquid. A typical dose of cimetidine might be 200 mg given at bedtime. A typical dosing of coated or uncoated digestive enzymes for patients over the age of 16 in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adult patients is 20-1600 mg at bedtime in tablet or liquid. A typical dose of cimetidine might be 200 mg given at bedtime. A typical dosing of coated or uncoated digestive enzymes for patients over the age of 16 in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of diabetic cardiovascular neuropathy in adult patients is 20-1600 mg at bedtime in tablet or liquid. A typical dose of cimetidine might be 200 mg given at bedtime. A typical dosing of coated or uncoated digestive enzymes for patients over the age of 16 in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Complex Regional Pain Syndrome in adult patients is 20-1600 mg at bedtime in tablet or liquid. A typical dose of cimetidine might be 200 mg given at bedtime. A typical dosing of coated or uncoated digestive enzymes for patients over the age of 16 in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Alzheimer's Disease in adult patients is 20-1600 mg at bedtime in tablet or liquid. A typical dose of cimetidine might be 200 mg given at bedtime. A typical dosing of coated or uncoated digestive enzymes for patients over the age of 16 in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Bipolar Disorder, Obsessive Compulsive Disorder or Oppositional Defiant Disorder in adult patients is 20-1600 mg at bedtime in tablet or liquid. A typical dose of cimetidine might be 200 mg given at bedtime. A typical dosing of coated or uncoated digestive enzymes for patients over the age of 16 in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of symptoms of addiction in adult patients is 20-1600 mg at bedtime in tablet or liquid. A typical dose of cimetidine might be 200 mg given at bedtime. A typical dosing of coated or uncoated digestive enzymes for patients over the age of 16 in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of William's Syndrome in adult patients is 20-1600 mg at bedtime in tablet or liquid. A typical dose of cimetidine might be 200 mg given at bedtime. A typical dosing of coated or uncoated digestive enzymes for patients over the age of 16 in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Cystic Fibrosis in adult patients is 20-1600 mg at bedtime in tablet or liquid. A typical dose of cimetidine might be 200 mg given at bedtime. A typical start dosing of coated or uncoated digestive enzymes in combination with cimetidine is 2,500 U.S.P. units of lipase per kilogram three times per day for adults. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Prion Diseases in adult patients is 20-1600 mg at bedtime in tablet or liquid. A typical dose of cimetidine might be 200 mg given at bedtime. A typical dosing of coated or uncoated digestive enzymes in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage

One example of a formulation of coated or uncoated PEC in combination with cimetidine for the treatment of Autism, ADD, ADHD and other pervasive developmental disorders in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Parkinson's in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage

One example of a formulation of coated or uncoated PEC in combination with cimetidine for the treatment of Familial Dysautonomia in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Guillain-Barre Syndrome in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is also present if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of neuroblastoma in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of diabetic cardiovascular neuropathy in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Complex Regional Pain Syndrome in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Alzheimer's Disease in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Bipolar Disorder, Obsessive Compulsive Disorder and Oppositional Defiant Disorder in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of symptoms of addiction in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of William's Syndrome in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Cystic Fibrosis in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical starting dosing of coated or uncoated digestive enzymes in combination with cimetidine for cystic fibrosis is 2,500 U.S.P. units of lipase per kilogram three times per day in patients ranging in age from 4 years and older. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with cimetidine for the treatment of Prion Diseases in patients with impaired renal function is 20-300 mg every 12 hours. The frequency of dosing may be increased to every 8 hours with caution. The dosing schedule should also be adjusted to coincide with the end of hemodialysis as cimetidine is cleared by hemodialysis. Further dosage reduction is necessary if liver impairment is also present. A typical dose of cimetidine might be 200 mg given at bedtime. A typical dosing of coated or uncoated digestive enzymes for patients in combination with cimetidine is 4,300 U.S.P. units of protease per kilogram three times per day. Dosing for both the enzyme composition or enzyme preparation and cimetidine may be from the minimal clinically proven safe and efficacious dosing to the maximal proven safe and efficacious dosage.

One example of a formulation of coated or uncoated PEC in combination with omeprazole for the treatment of Autism, ADD, ADHD and other pervasive developmental disorders in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical dosing of coated or uncoated digestive enzymes for patients over 2 years of age in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Familial Dysautonomia in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical dosing of coated or uncoated digestive enzymes for patients over 2 years of age in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Guillain-Barre Syndrome in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical dosing of coated or uncoated digestive enzymes for patients over 2 years of age in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of neuroblastoma in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical dosing of coated or uncoated digestive enzymes for patients over 2 years of age in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical dosing of coated or uncoated digestive enzymes for patients over 2 years of age in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of diabetic cardiovascular neuropathy in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical dosing of coated or uncoated digestive enzymes for patients over 2 years of age in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Complex Regional Pain Syndrome in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical dosing of coated or uncoated digestive enzymes for patients over 2 years of age in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Bipolar Disorder, Obsessive Compulsive Disorder or Oppositional Defiant Disorder in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical dosing of coated or uncoated digestive enzymes for patients over 2 years of age in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of symptoms of addiction in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical dosing of coated or uncoated digestive enzymes for patients over 2 years of age in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of William's Syndrome in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical dosing of coated or uncoated digestive enzymes for patients over 2 years of age in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Cystic Fibrosis in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical start dosing of coated or uncoated digestive enzymes in combination with nizatidine is 2,500 U.S.P. units of lipase per kilogram three times per day for children older than 4 years. A typical starting dose of coated or uncoated digestive enzymes in children older than 12 months but younger than 4 years in 1,000 Lipase USP per kilogram. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Prion Diseases in children over 2 years of age and weighing less than 20 kg is 0.2-10 mg/day in capsule, tablet or by granules for suspension. In children over 2 years of age and weighing over 20 kg the dose is 0.2-20 mg/day in capsule, tablet or by granules for suspension. A typical dose of omeprazole might be adding the contents of a 10 mg packet of granules to the first daily dose of PEC. A typical dosing of coated or uncoated digestive enzymes for patients over 2 years of age in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Autism, ADD, ADHD and other pervasive developmental disorders in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Familial Dysautonomia in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Guillain-Barre Syndrome in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of neuroblastoma in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of diabetic cardiovascular neuropathy in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Complex Regional Pain Syndrome in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Bipolar Disorder, Obsessive Compulsive Disorder or Oppositional Defiant Disorder in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of symptoms of addiction in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of William's Syndrome in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Cystic Fibrosis in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 2,500 U.S.P. units of lipase per kilogram three times per day. Dosing for both the enzyme composition or enzyme preparation and omeprazole may be from the minimal clinically proven safe and efficacious dosing to the maximal proven safe and efficacious dosage

One example of a formulation of coated or uncoated PEC in combination with omeprazole for the treatment of Prion Diseases in adolescents is 1-40 mg/day in tablet, capsule or by granules for suspension. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Autism, ADD, ADHD and other pervasive developmental disorders in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes for adolescent patients in combination with omeprazole is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Parkinson's Disease in adults is 1-40 mg/day in tablet, capsule or by granules for suspension. Dosage reduction may be necessary in patients with hepatic impairment. A typical dose of omeprazole might be adding the contents of a 1 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Familial Dysautonomia in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Guillain-Barre Syndrome in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of neuroblastoma in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of diabetic cardiovascular neuropathy in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Complex Regional Pain Syndrome in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Alzheimer's Disease in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Bipolar Disorder, Obsessive Compulsive Disorder or Oppositional Defiant Disorder in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of symptoms of addiction in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of William's Syndrome in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Cystic Fibrosis in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 2,500 U.S.P. units of lipase per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole for the treatment of Prion Diseases in adults is 1-40 mg/day in tablet, capsule or by granules for suspension. Dosage reduction may be necessary in patients with hepatic impairment. A typical dose of omeprazole might be adding the contents of a 20 mg packet of granules to the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole may be from the minimal clinically proven safe and efficacious dosing to the maximal proven safe and efficacious dosage.

One example of a formulation of coated or uncoated PEC in combination with omeprazole/sodium bicarbonate for the treatment of Autism, ADD, ADHD and other pervasive developmental disorders in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of Parkinson's Disease in adults over the age of 18 years is 2-40/11-1680 mg/mg/day in capsule of by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of Familial Dysautonomia in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of Guillain-Barre Syndrome in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of neuroblastoma in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults over the age of 18 years is 1-40/55-1680 mg/mg/day in capsule of by powder for suspension. A typical dose of omeprazole/sodium bicarbonate might be 20/1100 mg/mg/day given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of diabetic cardiovascular neuropathy in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of Complex Regional Pain Syndrome in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of Alzheimer's Disease in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of Bipolar Disorder, Obsessive Compulsive Disorder or Oppositional Defiant Disorder in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of symptoms of addiction in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of William's Syndrome in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of Cystic Fibrosis in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 2,500 U.S.P. units of lipase per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with omeprazole/sodium bicarbonate for the treatment of Prion Diseases in adults over the age of 18 years is 2-40/110-1680 mg/mg/day in capsule or by powder for suspension. A typical dose of omeprazole/sodium bicarbonate might be 20/1100 mg/mg given with the first daily dose of PEC. A typical dosing of coated or uncoated digestive enzymes in combination with omeprazole/sodium bicarbonate for patients over the age of 16 is 4,300 U.S.P. units of protease per kilogram three times per day. Dosing for both the enzyme composition or enzyme preparation and omeprazole/sodium bicarbonate may be from the minimal clinically proven safe and efficacious dosing to the maximal proven safe and efficacious dosage.

One example of a formulation of coated or uncoated PEC in combination with esomeprazole for the treatment of Autism, ADD, ADHD and other pervasive developmental disorders in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Familial Dysautonomia in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Guillain-Barre Syndrome in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of neuroblastoma in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of diabetic cardiovascular neuropathy in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Complex Regional Pain Syndrome in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Bipolar Disorder, Obsessive Compulsive Disorder or Oppositional Defiant Disorder in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of symptoms of addiction in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of William's Syndrome in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Cystic Fibrosis in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 2,500 U.S.P. units of lipase per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Prion Diseases in patients ranging in age from 1 to 11 years and weighing less than 20 kg is 0.1-10 mg/day in capsule or by powder for suspension. In patients ranging in age from 1 to 11 years and weighing over 20 kg the dose is 0.1-20 mg/day in capsule or by powder for suspension. A typical dose of esomeprazole might be adding the contents of a 10 mg packet to the first daily dose of PEC. A typical dosing of coated or uncoated digestive enzymes in combination with esomeprazole for patients from 1 to 11 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Autism, ADD, ADHD and other pervasive developmental disorders in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Familial Dysautonomia in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Guillain-Barre Syndrome in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of neuroblastoma in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of diabetic cardiovascular neuropathy in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Complex Regional Pain Syndrome in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Bipolar Disorder, Obsessive Compulsive Disorder or Oppositional Defiant Disorder in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of symptoms of addiction in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of William's Syndrome in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Cystic Fibrosis in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 2,500 U.S.P. units of lipase per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Prion Diseases in patients ranging in age from 12 to 17 years is 1-40 mg/day in capsule or by powder for suspension. A typical dose of esomeprazole might be adding the contents of a 20 mg packet to the first daily dose of PEC. A typical dosing of coated or uncoated digestive enzymes in combination with esomeprazole for patients from 12 to 17 years is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Autism, ADD, ADHD and other pervasive developmental disorders in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Parkinson's Disease in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Familial Dysautonomia in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Guillain-Barre Syndrome in adults is 1-40 mg/day in capsule or by powder for suspension. Patients with severe hepatic insufficiency (Child Pugh Class C) must not exceed 20 mg/day. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of neuroblastoma in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of diabetic cardiovascular neuropathy in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Complex Regional Pain Syndrome in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Alzheimer's Disease in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Bipolar Disorder, Obsessive Compulsive Disorder or Oppositional Defiant Disorder in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of symptoms of addiction in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of William's Syndrome in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Cystic Fibrosis in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 2,500 U.S.P. units of lipase per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with esomeprazole for the treatment of Prion Diseases in adults is 1-40 mg/day in capsule or by powder for suspension. Patients with severe hepatic insufficiency (Child Pugh Class C) must not exceed 20 mg/day. A typical dose of esomeprazole might be 20 mg taken with the first daily dose of PEC. A typical dosing of coated or uncoated digestive enzymes in combination with esomeprazole for patients over 16 years of age is 4,300 U.S.P. units of protease per kilogram three times per day. Dosing for both the enzyme composition or enzyme preparation and esomeprazole may be from the minimal clinically proven safe and efficacious dosing to the maximal proven safe and efficacious dosage.

One example of a formulation of coated or uncoated PEC in combination with lansoprazole for the treatment of Autism, ADD, ADHD and other pervasive developmental disorders in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Familial Dysautonomia in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Guillain-Barre Syndrome in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of neuroblastoma in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of diabetic cardiovascular neuropathy in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Complex Regional Pain Syndrome in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Bipolar Disorder, Obsessive Compulsive Disorder or Oppositional Defiant Disorder in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of symptoms of addiction in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of William's Syndrome in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Cystic Fibrosis in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 2,500 U.S.P. units of lipase per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Prion Diseases in children weighing less than 30 kg is 0.5-15 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage

One example of a formulation of coated or uncoated PEC in combination with lansoprazole for the treatment of Autism, ADD, ADHD and other pervasive developmental disorders in children weighing over 30 kg is 1.5-60 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Familial Dysautonomia in children weighing over 30 kg is 1.5-60 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Guillain-Barre Syndrome in children weighing over 30 kg is 1.5-60 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of neuroblastoma in children weighing over 30 kg is 1.5-60 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in children weighing over 30 kg is 1.5-60 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of diabetic cardiovascular neuropathy in children weighing over 30 kg is 1.5-60 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Complex Regional Pain Syndrome in children weighing over 30 kg is 1.5-60 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Bipolar Disorder, Obsessive Compulsive Disorder or Oppositional Defiant Disorder in children weighing over 30 kg is 1.5-60 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of symptoms of addiction in children weighing over 30 kg is 1.5-60 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of William's Syndrome in children weighing over 30 kg is 1.5-60 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Cystic Fibrosis in children weighing over 30 kg is 1.5-60 mg/day in capsule, SoluTab™ or by granules for suspension. A typical dose of lansoprazole might be administering a 15 mg SoluTab™ just prior to the first daily dose of PEC. A typical dosing of coated or uncoated digestive enzymes in combination with lansoprazole for children is 2,500 U.S.P. units of lipase per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Prion Diseases in children weighing over 30 kg is 1.5-60 mg/day in capsule, SoluTab™ or by granules for suspension. A typical dose of lansoprazole might be administering a 15 mg SoluTab just prior to the first daily dose of PEC. A typical dosing of coated or uncoated digestive enzymes in combination with lansoprazole for children is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Autism, ADD, ADHD and other pervasive developmental disorders in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Parkinson's Disease in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Familial Dysautonomia in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Guillain-Barre Syndrome in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of neuroblastoma in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of diabetic cardiovascular neuropathy in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Complex Regional Pain Syndrome in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Alzheimer's Disease in adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adults is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Bipolar Disorder, Obsessive Compulsive Disorder or Oppositional Defiant Disorder in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents and adults is 4,300 U.S.P. units of protease per kilogram three times per day.Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of symptoms of addiction in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents and adults is 4,300 U.S.P. units of protease per kilogram three times per day.Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of William's Syndrome in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents and adults is 4,300 U.S.P. units of protease per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Cystic Fibrosis in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents and adults is 2,500 U.S.P. units of lipase per kilogram three times per day. Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with lansoprazole for the treatment of Prion Diseases in adolescents and adults is 1.5-90 mg/day in capsule, SoluTab™ or by granules for suspension. Dosage reduction should be considered in patients with severe hepatic disease. A typical dose of lansoprazole might be 30 mg taken immediately prior to the first daily dose of PEC. A typical dosing of coated or uncoated digestive enzymes in combination with lansoprazole for adolescents and adults is 4,300 U.S.P. units of protease per kilogram three times per day.Dosing for both the enzyme composition or enzyme preparation and lansoprazole may be from the minimal clinically proven safe and efficacious dosing to the maximal proven safe and efficacious dosage.

One example of a formulation of coated or uncoated PEC in combination with dexlansoprazole for the treatment of Autism, ADD, ADHD and other pervasive developmental disorders in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of Parkinson's Disease in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of Familial Dysautonomia in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of Guillain-Barre Syndrome in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of neuroblastoma in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of diabetic cardiovascular neuropathy in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of Complex Regional Pain Syndrome in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of Alzheimer's Disease in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of Bipolar Disorder, Obsessive Compulsive Disorder or Oppositional Defiant Disorder in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of symptoms of addiction in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of William's Syndrome in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of Cystic Fibrosis in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 2,500 U.S.P. units of lipase per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with dexlansoprazole for the treatment of Prion Diseases in adults is 3-60 mg/day in capsule form. Patients with moderate hepatic impairment (Child Pugh Class B) should not exceed 30 mg/day. A typical dose of dexlansoprazole might be 15 mg taken immediately prior to the first daily dose of PEC. A typical dosing of coated or uncoated digestive enzymes in combination with dexlansoprazole for adults is 4,300 U.S.P. units of protease per kilogram three times per day. Dosing for both the enzyme composition or enzyme preparation and dexlansoprazole may be from the minimal clinically proven safe and efficacious dosing to the maximal proven safe and efficacious dosage.

One example of a formulation of coated or uncoated PEC in combination with pantoprazole for the treatment of Autism, ADD, ADHD and other pervasive developmental disorders in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of Parkinson's Disease in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of Familial Dysautonomia in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of Guillain-Barre Syndrome in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of neuroblastoma in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of diabetic cardiovascular neuropathy in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of Complex Regional Pain Syndrome in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of Alzheimer's Disease in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of Bipolar Disorder, Obsessive Compulsive Disorder or Oppositional Defiant Disorder in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of symptoms of addiction in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of William's Syndrome in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of Cystic Fibrosis in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 2,500 U.S.P. units of lipase per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with pantoprazole for the treatment of Prion Diseases in adults is 1-80 mg/day in tablet or by granules for suspension. A typical dose of pantoprazole might be 30 mg taken immediately prior to the first daily dose of PEC. A typical dosing of coated or uncoated digestive enzymes in combination with pantoprazole for adults is 4,300 U.S.P. units of protease per kilogram three times per day. Dosing for both the enzyme composition or enzyme preparation and pantoprazole may be from the minimal clinically proven safe and efficacious dosing to the maximal proven safe and efficacious dosage.

One example of a formulation of coated or uncoated PEC in combination with rabeprazole for the treatment of Autism, ADD, ADHD and other pervasive developmental disorders in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Familial Dysautonomia in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Guillain-Barre Syndrome in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of neuroblastoma in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of diabetic cardiovascular neuropathy in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Complex Regional Pain Syndrome in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Bipolar Disorder, Obsessive Compulsive Disorder or Oppositional Defiant Disorder in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of symptoms of addiction in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of William's Syndrome in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Cystic Fibrosis in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 2,500 U.S.P. units of lipase per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Prion Diseases in adolescents is 0.2-20 mg/day in tablet form. A typical dose of rabeprazole might be 20 mg given immediately prior to the first daily dose of PEC. A typical dosing of coated or uncoated digestive enzymes in combination with rabeprazole for adolescents is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Autism, ADD, ADHD and other pervasive developmental disorders in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Parkinson's Disease in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Familial Dysautonomia in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Guillain-Barre Syndrome in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of neuroblastoma in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults is 1-40 mg/day in tablet form. Caution should be used in dosing for patients with sever hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of diabetic cardiovascular neuropathy in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Complex Regional Pain Syndrome in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Alzheimer's Disease in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Bipolar Disorder, Obsessive Compulsive Disorder or Oppositional Defiant Disorder in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of symptoms of addiction in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of William's Syndrome in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Cystic Fibrosis in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 2,500 U.S.P. units of lipase per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with rabeprazole for the treatment of Prion Diseases in adults is 1-40 mg/day in tablet form. Caution should be used in dosing for patients with severe hepatic impairment. A typical dose of rabeprazole might be 20 mg given immediately prior to the first daily dose of PEC. A typical dosing of coated or uncoated digestive enzymes in combination with rabeprazole for adults is 4,300 U.S.P. units of protease per kilogram three times per day. Dosing for both the enzyme composition or enzyme preparation and rabeprazole may be from the minimal clinically proven safe and efficacious dosing to the maximal proven safe and efficacious dosage.

One example of a formulation of coated or uncoated PEC in combination with sucralfate for the treatment of Autism, ADD, ADHD and other pervasive developmental disorders in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 4,300 U.S.P. units of protease per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with sucralfate for the treatment of Familial Dysautonomia in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 4,300 U.S.P. units of protease per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with sucralfate for the treatment of Guillain-Barre Syndrome in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 4,300 U.S.P. units of protease per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with sucralfate for the treatment of neuroblastoma in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 4,300 U.S.P. units of protease per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with sucralfate for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, diabetic autonomic failure, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 4,300 U.S.P. units of protease per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with sucralfate for the treatment of diabetic cardiovascular neuropathy in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 4,300 U.S.P. units of protease per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with sucralfate for the treatment of Complex Regional Pain Syndrome in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 4,300 U.S.P. units of protease per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with sucralfate for the treatment of Bipolar Disorder, Obsessive Compulsive Disorder or Oppositional Defiant Disorder in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 4,300 U.S.P. units of protease per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with sucralfate for the treatment of symptoms of addiction in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 4,300 U.S.P. units of protease per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with sucralfate for the treatment of William's Syndrome in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 4,300 U.S.P. units of protease per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with sucralfate for the treatment of Cystic Fibrosis in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 2,500 U.S.P. units of lipase per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with sucralfate for the treatment of Prion Diseases in children is 1-80 mg/kg/day or up to 2000 mg/day in tablet or suspension. A typical dose of sucralfate might be 500 mg immediately preceding each dose of PEC. A typical dosing of coated or uncoated digestive enzymes in combination with sucralfate for children is 4,300 U.S.P. units of protease per kilogram three times per day. Dosing for both the enzyme composition or enzyme preparation and sucralfate may be from the minimal clinically proven safe and efficacious dosing to the maximal proven safe and efficacious dosage.

One example of a formulation of coated or uncoated PEC in combination with bismuth subsalicylate for the treatment of Autism, ADD, ADHD and other pervasive developmental disorders in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of Parkinson's Disease in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of Familial Dysautonomia in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of Guillain-Barre Syndrome in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of neuroblastoma in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of diabetic cardiovascular neuropathy in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of Complex Regional Pain Syndrome in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of Alzheimer's Disease in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of Bipolar Disorder, Obsessive Compulsive Disorder or Oppositional Defiant Disorder in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of symptoms of addiction in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of William's Syndrome in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of Cystic Fibrosis in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 2,500 U.S.P. units of lipase per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with bismuth subsalicylate for the treatment of Prion Diseases in patients over 12 years is 0.1-4200 mg/day in caplet, chewable tablet or suspension. A typical dose of bismuth subsalicylate might be chewing a 262 mg tablet prior to each dose of PEC. A typical dosing of coated or uncoated digestive enzymes in combination with bismuth subsalicylate for patients over 12 years of age is 4,300 U.S.P. units of protease per kilogram three times per day. Dosing for both the enzyme composition or enzyme preparation and bismuth subsalicylate may be from the minimal clinically proven safe and efficacious dosing to the maximal proven safe and efficacious dosage.

One example of a formulation of coated or uncoated PEC in combination with metoclopramide for the treatment of Autism, ADD, ADHD and other pervasive developmental disorders in adult patients is 0.1-15 mg up to four times daily in tablet, orally-disintegrating tablet or oral solution. A typical dose of metoclopramide might be 10 mg given immediately prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Parkinson's in adult patients is 0.1-15 mg up to four times daily in tablet, orally-disintegrating tablet or oral solution. A typical dose of metoclopramide might be 10 mg given immediately prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Familial Dysautonomia in adult patients is 0.1-15 mg up to four times daily in tablet, orally-disintegrating tablet or oral solution. A typical dose of metoclopramide might be 10 mg given immediately prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Guillain-Barre Syndrome in adult patients is 0.1-15 mg up to four times daily in tablet, orally-disintegrating tablet or oral solution. A typical dose of metoclopramide might be 10 mg given immediately prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of neuroblastoma in adult patients is 0.1-15 mg up to four times daily in tablet, orally-disintegrating tablet or oral solution. A typical dose of metoclopramide might be 10 mg given immediately prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menkes disease, MAO deficiency states, Chagas disease, pure autonomic failure, syncope, hypertension, cardiovascular disease, and renal disease) in adult patients is 0.1-15 mg up to four times daily in tablet, orally-disintegrating tablet or oral solution. A typical dose of metoclopramide might be 10 mg given immediately prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of diabetic cardiovascular neuropathy in adult patients is 0.1-15 mg up to four times daily in tablet, orally-disintegrating tablet or oral solution. A typical dose of metoclopramide might be 10 mg given immediately prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Complex Regional Pain Syndrome in adult patients is 0.1-15 mg up to four times daily in tablet, orally-disintegrating tablet or oral solution. A typical dose of metoclopramide might be 10 mg given immediately prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Bipolar Disorder, Obsessive Compulsive Disorder or Oppositional Defiant Disorder in adult patients is 0.1-15 mg up to four times daily in tablet, orally-disintegrating tablet or oral solution. A typical dose of metoclopramide might be 10 mg given immediately prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of symptoms of addiction in adult patients is 0.1-15 mg up to four times daily in tablet, orally-disintegrating tablet or oral solution. A typical dose of metoclopramide might be 10 mg given immediately prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of William's Syndrome in adult patients is 0.1-15 mg up to four times daily in tablet, orally-disintegrating tablet or oral solution. A typical dose of metoclopramide might be 10 mg given immediately prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Cystic Fibrosis in adult patients is 0.1-15 mg up to four times daily in tablet, orally-disintegrating tablet or oral solution. A typical dose of metoclopramide might be 10 mg given 30 minutes prior to the first PEC administration of the day. A typical dosing of coated or uncoated digestive enzymes in combination with metoclopramide for adult patients is 2,500 U.S.P. units of lipase per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Prion Diseases in adult patients is 0.1-15 mg up to four times daily in tablet, orally-disintegrating tablet or oral solution. A typical dose of metoclopramide might be 10 mg given 30 minutes prior to each PEC administration. A typical dosing of coated or uncoated digestive enzymes in combination with metoclopramide for adult patients is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Autism, ADD, ADHD and other pervasive developmental disorders in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Parkinson's in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Familial Dysautonomia in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Guillain-Barre Syndrome in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of neuroblastoma in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menkes disease, MAO deficiency states, Chagas disease, pure autonomic failure, syncope, hypertension, cardiovascular disease, and renal disease) in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of diabetic cardiovascular neuropathy in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Complex Regional Pain Syndrome in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Alzheimer's Disease in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Bipolar Disorder, Obsessive Compulsive Disorder or Oppositional Defiant Disorder in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of symptoms of addiction in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of William's Syndrome in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Cystic Fibrosis in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 2,500 U.S.P. units of lipase per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with metoclopramide for the treatment of Prion Diseases in elderly or patients with impaired renal function (creatinine clearance <40 mL/min) is 0.05-10 mg up to four times daily in tablet, orally-disintegrating tablet or oral solution. A typical dose of metoclopramide might be 5 mg given immediately prior to each PEC administration. A typical dosing of coated or uncoated digestive enzymes in combination with metoclopramide is 4,300 U.S.P. units of protease per kilogram three times per day. Dosing for both the enzyme composition or enzyme preparation and metoclopramide may be from the minimal clinically proven safe and efficacious dosing to the maximal proven safe and efficacious dosage.

One example of a formulation of coated or uncoated PEC in combination with bethanecol for the treatment of Autism, ADD, ADHD and other pervasive developmental disorders in adult patients is 0.1-50 mg up to four times daily in tablet. A typical dose of bethanecol might be 25 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of Parkinson's in adult patients is 0.1-50 mg up to four times daily in tablet. A typical dose of bethanecol might be 25 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of Familial Dysautonomia in adult patients is 0.1-50 mg up to four times daily in tablet. A typical dose of bethanecol might be 25 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of Guillain-Barre Syndrome in adult patients is 0.1-50 mg up to four times daily in tablet. A typical dose of bethanecol might be 25 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of neuroblastoma in adult patients is 0.1-50 mg up to four times daily in tablet. A typical dose of bethanecol might be 25 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menkes disease, MAO deficiency states, Chagas disease, pure autonomic failure, syncope, hypertension, cardiovascular disease, and renal disease) in adult patients is 0.1-50 mg up to four times daily in tablet. A typical dose of bethanecol might be 25 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of diabetic cardiovascular neuropathy in adult patients is 0.1-50 mg up to four times daily in tablet. A typical dose of bethanecol might be 25 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of Complex Regional Pain Syndrome in adult patients is 0.1-50 mg up to four times daily in tablet. A typical dose of bethanecol might be 25 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of Bipolar Disorder, Obsessive Compulsive Disorder or Oppositional Defiant Disorder in adult patients is 0.1-50 mg up to four times daily in tablet. A typical dose of bethanecol might be 25 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of symptoms of addiction in adult patients is 0.1-50 mg up to four times daily in tablet. A typical dose of bethanecol might be 10 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of William's Syndrome in adult patients is 0.1-50 mg up to four times daily in tablet. A typical dose of bethanecol might be 25 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of Cystic Fibrosis in adult patients is 0.1-50 mg up to four times daily in tablet. A typical dose of bethanecol might be 25 mg given one hour prior to the first PEC administration of the day. A typical dosing of coated or uncoated digestive enzymes in combination with bethanecol for adult patients is 2,500 U.S.P. units of lipase per kilogram three times per day. Dosing for both the enzyme composition or enzyme preparation and bethanecol may be from the minimal clinically proven safe and efficacious dosing to the maximal proven safe and efficacious dosage.

One example of a formulation of coated or uncoated PEC in combination with bethanecol for the treatment of Prion Diseases in adult patients is 0.1-50 mg up to four times daily in tablet. A typical dose of bethanecol might be 25 mg given one hour prior to each PEC administration. A typical dosing of coated or uncoated digestive enzymes in combination with bethanecol for adult patients is 4,300 U.S.P. units of protease per kilogram three times per day. Dosing for both the enzyme composition or enzyme preparation and bethanecol may be from the minimal clinically proven safe and efficacious dosing to the maximal proven safe and efficacious dosage.

One example of a formulation of uncoated PEC in combination with scopolamine for the treatment of Autism, ADD, ADHD and other pervasive developmental disorders in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Familial Dysautonomia in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Guillain-Barre Syndrome in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of neuroblastoma in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of diabetic cardiovascular neuropathy in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Complex Regional Pain Syndrome in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Bipolar Disorder, Obsessive Compulsive Disorder or Oppositional Defiant Disorder in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of the symptoms of addiction in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of William's Syndrome in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Cystic Fibrosis in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2,500 U.S.P. units of lipase per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Prion Diseases in patients ranging in age from 6 months to 3 years is 0.0006 mg/kg up to a max of 0.15 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Autism, ADD, ADHD and other pervasive developmental disorders in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine for patients ranging in age from 6 months to 3 years is 2600 U.S.P. units of protease per kilogram three times per day. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Familial Dysautonomia in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine is 4,300 U.S.P. units of protease per kilogram three times per day in patients ranging in age from 3 to 16 years of age. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Guillain-Barre Syndrome in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dose of scopolamine might be 0.1 mg given orally once daily. A typical dosing of coated or uncoated digestive enzymes in combination with scopolamine is 4,300 U.S.P. units of protease per kilogram three times per day in patients ranging in age from 3 to 12 years of age. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of neuroblastoma in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dose of scopolamine might be 0.1 mg given orally once daily. A typical dosing of coated or uncoated digestive enzymes in combination with scopolamine is 4,300 U.S.P. units of protease per kilogram three times per day in patients ranging in age from 3 to 12 years of age. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dose of scopolamine might be 0.1 mg given orally once daily. A typical dosing of coated or uncoated digestive enzymes in combination with scopolamine is 4,300 U.S.P. units of protease per kilogram three times per day in patients ranging in age from 3 to 12 years of age. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of diabetic cardiovascular neuropathy in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine is 4,300 U.S.P. units of protease per kilogram three times per day in patients ranging in age from 3 to 12 years of age. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Complex Regional Pain Syndrome in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine is 4,300 U.S.P. units of protease per kilogram three times per day in patients ranging in age from 3 to 12 years of age. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Bipolar Disorder, Obsessive Compulsive Disorder or Oppositional Defiant Disorder in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine is 4,300 U.S.P. units of protease per kilogram three times per day in patients ranging in age from 3 to 12 years of age. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of the symptoms of addiction in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine is 4,300 U.S.P. units of protease per kilogram three times per day in patients ranging in age from 3 to 12 years of age. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of William's Syndrome in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine is 4,300 U.S.P. units of protease per kilogram three times per day in patients ranging in age from 3 to 12 years of age. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Cystic Fibrosis in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine is 2,500 U.S.P. units of lipase per kilogram three times per day in patients ranging in age from 3 to 12 years of age. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Prion Diseases in patients ranging in age from 3 to 12 years is 0.0006 mg/kg up to a max of 0.3 mg/day in soluble tablet, intravenous, intramuscular or subcutaneous forms. A typical dose of scopolamine might be 0.1 mg given orally once daily. A typical dosing of coated or uncoated digestive enzymes in combination with scopolamine is 4,300 U.S.P. units of protease per kilogram three times per day in patients ranging in age from 3 to 12 years of age. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Autism, ADD, ADHD and other pervasive developmental disorders in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 4,300 U.S.P. units of protease per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Familial Dysautonomia in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 4,300 U.S.P. units of protease per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Guillain-Barre Symdrome in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 4,300 U.S.P. units of protease per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of neuroblastoma in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 4,300 U.S.P. units of protease per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 4,300 U.S.P. units of protease per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of diabetic cardiovascular neuropathy in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 4,300 U.S.P. units of protease per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Complex Regional Pain Syndrome in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 4,300 U.S.P. units of protease per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Bipolar Disorder, Obsessive Compulsive Disorder or Oppositional Defiant Disorder in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 4,300 U.S.P. units of protease per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of symptoms of addiction in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 4,300 U.S.P. units of protease per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of William's Syndrome in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 4,300 U.S.P. units of protease per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Cystic Fibrosis in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 2,500 U.S.P. units of lipase per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with scopolamine for the treatment of Prion Diseases in adults is 0.032 - 0.65 mg intravenously, intramuscularly or subcutaneously, 0.04 - 0.8 mg orally, or by the typical application and subsequent removal of one patch behind an ear every 3 days. A typical dosing of coated or uncoated digestive enzymes in combination with scopolamine is 4,300 U.S.P. units of protease per kilogram three times per day for adult patients. Dosing for both the enzyme composition or enzyme preparation and scopolamine may be from the minimal clinically proven safe and efficacious dosing to the maximal proven safe and efficacious dosage.

One example of a formulation of uncoated PEC in combination with trihexyphenidyl for the treatment of Autism, ADD, ADHD and other pervasive developmental disorders in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 4,300 U.S.P. units of protease per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with trihexyphenidyl for the treatment of Familial Dysautonomias in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 4,300 U.S.P. units of protease per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with trihexyphenidyl for the treatment of Guillain-Barre Syndrome in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 4,300 U.S.P. units of protease per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with trihexyphenidyl for the treatment of neuroblastoma in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 4,300 U.S.P. units of protease per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with trihexyphenidyl for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 4,300 U.S.P. units of protease per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with trihexyphenidyl for the treatment of diabetic cardiovascular neuropathy in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 4,300 U.S.P. units of protease per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with trihexyphenidyl for the treatment of Complex Regional Pain Syndrome in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 4,300 U.S.P. units of protease per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with trihexyphenidyl for the treatment of Bipolar Disorder, Obsessive Compulsive Disorder or Oppositional Defiant Disorder in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 4,300 U.S.P. units of protease per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with trihexyphenidyl for the treatment of symptoms of addiction in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 4,300 U.S.P. units of protease per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with trihexyphenidyl for the treatment of William's Syndrome in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 4,300 U.S.P. units of protease per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with trihexyphenidyl for the treatment of Cystic Fibrosis in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 2,500 U.S.P. units of lipase per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with trihexyphenidyl for the treatment of Prion Diseases in adults is 0.6 to 10 mg/day in three or four divided doses. A typical dose of trihexyphenidyl might be 2 mg three times daily with a meal in tablet or elixir. A typical dosing of coated or uncoated digestive enzymes in combination with trihexyphenidyl is 4,300 U.S.P. units of protease per kilogram three times per day for adult patients. Dosing for both the enzyme composition or enzyme preparation and trihexyphenidyl may be from the minimal clinically proven safe and efficacious dosing to the maximal proven safe and efficacious dosage.

One example of a formulation of uncoated PEC in combination with benztropine for the treatment of Autism, ADD, ADHD and other pervasive developmental disorders in adults is 0.05 to 8 mg/day in two divided doses. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with benztropine for the treatment of Familial Dysautonomias in adults is 0.05 to 8 mg/day in two divided doses. A typical dose of benztropine might be 1 mg twice daily in tablet or injection. Dosage reduction may be necessary in patients over the age of 60. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with benztropine for the treatment of Guillain-Barre Syndrome in adults is 0.05 to 8 mg/day in two divided doses. A typical dose of benztropine might be 1 mg twice daily in tablet or injection. Dosage reduction may be necessary in patients over the age of 60. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with benztropine for the treatment of neuroblastoma in adults is 0.05 to 8 mg/day in two divided doses. A typical dose of benztropine might be 1 mg twice daily in tablet or injection. Dosage reduction may be necessary in patients over the age of 60. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with benztropine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults is 0.05 to 8 mg/day in two divided doses. A typical dose of benztropine might be 1 mg twice daily in tablet or injection. Dosage reduction may be necessary in patients over the age of 60. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with benztropine for the treatment of diabetic cardiovascular neuropathy in adults is 0.05 to 8 mg/day in two divided doses. A typical dose of benztropine might be 1 mg twice daily in tablet or injection. Dosage reduction may be necessary in patients over the age of 60. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with benztropine for the treatment of Complex Regional Pain Syndrome in adults is 0.05 to 8 mg/day in two divided doses. A typical dose of benztropine might be 1 mg twice daily in tablet or injection. Dosage reduction may be necessary in patients over the age of 60. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with benztropine for the treatment of Bipolar Disorder, Obsessive Compulsive Disorder or Oppositional Defiant Disorder in adults is 0.05 to 8 mg/day in two divided doses. A typical dose of benztropine might be 1 mg twice daily in tablet or injection. Dosage reduction may be necessary in patients over the age of 60. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with benztropine for the treatment of symptoms of addiction in adults is 0.05 to 8 mg/day in two divided doses. A typical dose of benztropine might be 1 mg twice daily in tablet or injection. Dosage reduction may be necessary in patients over the age of 60. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with benztropine for the treatment of William's Syndrome in adults is 0.05 to 8 mg/day in two divided doses. A typical dose of benztropine might be 1 mg twice daily in tablet or injection. Dosage reduction may be necessary in patients over the age of 60. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with benztropine for the treatment of Cystic Fibrosis in adults is 0.05 to 8 mg/day in two divided doses. A typical dose of benztropine might be 1 mg twice daily in tablet or injection. Dosage reduction may be necessary in patients over the age of 60. A typical dosing of coated or uncoated digestive enzymes in combination with benztropine is 2,500 U.S.P. units of lipase per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with benztropine for the treatment of Prion Diseases in adults is 0.05 to 8 mg/day in two divided doses. A typical dose of benztropine might be 1 mg twice daily in tablet or injection. Dosage reduction may be necessary in patients over the age of 60. A typical dose of benztropine might be 1 mg twice daily in tablet or injection. Dosage reduction may be necessary in patients over the age of 60. A typical dosing of coated or uncoated digestive enzymes in combination with benztropine is 4,300 U.S.P. units of protease per kilogram three times per day for adult patients. Dosing for both the enzyme composition or enzyme preparation and benztropine may be from the minimal clinically proven safe and efficacious dosing to the maximal proven safe and efficacious dosage.

One example of a formulation of uncoated PEC in combination with dicyclomine for the treatment of Autism, ADD, ADHD and other pervasive developmental disorders in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 4,300 U.S.P. units of protease per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with dicyclomine for the treatment of Familial Dysautonomias in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 4,300 U.S.P. units of protease per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with dicyclomine for the treatment of Guillain-Barre Syndrome in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 4,300 U.S.P. units of protease per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with dicyclomine for the treatment of neuroblastoma in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 4,300 U.S.P. units of protease per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with dicyclomine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 4,300 U.S.P. units of protease per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with dicyclomine for the treatment of diabetic cardiovascular neuropathy in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 4,300 U.S.P. units of protease per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with dicyclomine for the treatment of Complex Regional Pain Syndrome in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 4,300 U.S.P. units of protease per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with dicyclomine for the treatment of Bipolar Disorder, Obsessive Compulsive Disorder or Oppositional Defiant Disorder in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 4,300 U.S.P. units of protease per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with dicyclomine for the treatment of symptoms of addiction in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 4,300 U.S.P. units of protease per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with dicyclomine for the treatment of William's Syndrome in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 4,300 U.S.P. units of protease per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with dicyclomine for the treatment of Cystic Fibrosis in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 2,500 U.S.P. units of lipase per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with dicyclomine for the treatment of Prion Diseases in adults is 1 to 160 mg/day in equally divided doses. A typical dose of dicyclomine might be 20 mg 30 minutes prior to meals in tablet, capsule, syrup or injection. Dosage reduction may be necessary in patients over the age of 60. A typical dosing of coated or uncoated digestive enzymes in combination with dicyclomine is 4,300 U.S.P. units of protease per kilogram three times per day for adult patients. Dosing for both the enzyme composition or enzyme preparation and dicyclomine may be from the minimal clinically proven safe and efficacious dosing to the maximal proven safe and efficacious dosage.

One example of a formulation of uncoated PEC in combination with glycopyrrolate for the treatment of Autism, ADD, ADHD and other pervasive developmental disorders in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 4,300 U.S.P. units of protease per kilogram three times per day for adults and children over 12 years old. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with glycopyrrolate for the treatment of Familial Dysautonomias in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 4,300 U.S.P. units of protease per kilogram three times per day for adults and children over 12 years of age. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with glycopyrrolate for the treatment of Guillain-Barre Syndrome in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 4,300 U.S.P. units of protease per kilogram three times per day for adults and children over 12 years of age. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with glycopyrrolate for the treatment of neuroblastoma in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 4,300 U.S.P. units of protease per kilogram three times per day for adults and children over 12 years of age. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with glycopyrrolate for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 4,300 U.S.P. units of protease per kilogram three times per day for adults and children over 12 years of age. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with glycopyrrolate for the treatment of diabetic cardiovascular neuropathy in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 4,300 U.S.P. units of protease per kilogram three times per day for adults and children over 12 years of age. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with glycopyrrolate for the treatment of Complex Regional Pain Syndrome in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 4,300 U.S.P. units of protease per kilogram three times per day for adults and children over 12 years of age. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with glycopyrrolate for the treatment of Bipolar Disorder, Obsessive Compulsive Disorder or Oppositional Defiant Disorder in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 4,300 U.S.P. units of protease per kilogram three times per day for adults and children over 12 years of age. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with glycopyrrolate for the treatment of symptoms of addiction in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 4,300 U.S.P. units of protease per kilogram three times per day for adults and children over 12 years of age. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with glycopyrrolate for the treatment of William's Syndrome in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 4,300 U.S.P. units of protease per kilogram three times per day for adults and children over 12 years of age. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with glycopyrrolate for the treatment of Cystic Fibrosis in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 2,500 U.S.P. units of lipase per kilogram three times per day for adults and children over 12 years of age. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with glycopyrrolate for the treatment of Prion Diseases in adults and children over 12 years is 0.01 to 6 mg/day in divided doses. A typical dose of glycoppyrrolate might be 1 mg two to three times daily in tablet form. Dosage reduction may be necessary in patients over the age of 60. A typical dosing of coated or uncoated digestive enzymes in combination with glycopyrrolate is 4,300 U.S.P. units of protease per kilogram three times per day for adults and children over 12 years of age. Dosing for both the enzyme composition or enzyme preparation and glycopyrrolate may be from the minimal clinically proven safe and efficacious dosing to the maximal proven safe and efficacious dosage.

One example of a formulation of uncoated PEC in combination with hyoscyamine for the treatment of Autism, ADD, ADHD and other pervasive developmental disorders in children under 2 years is 0.0025 to 0.00815 mg/kg orally every 4 hours if needed. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Familial Dysautonomia in children under 2 years is 0.0025 to 0.00815 mg/kg orally every 4 hours if needed. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Guillain-Barre Syndrome in children under 2 years is 0.0025 to 0.00815 mg/kg orally every 4 hours if needed. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of neuroblastoma in children under 2 years is 0.0025 to 0.00815 mg/kg orally every 4 hours if needed. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in children under 2 years is 0.0025 to 0.00815 mg/kg orally every 4 hours if needed. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of diabetic cardiovascular neuropathy in children under 2 years is 0.0025 to 0.00815 mg/kg orally every 4 hours if needed. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Complex Regional Pain Syndrome in children under 2 years is 0.0025 to 0.00815 mg/kg orally every 4 hours if needed. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Bipolar Disorder, Obsessive Compulsive Disorder or Oppositional Defiant Disorder in children under 2 years is 0.0025 to 0.00815 mg/kg orally every 4 hours if needed. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of the symptoms of addiction in children under 2 years is 0.0025 to 0.00815 mg/kg orally every 4 hours if needed. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of William's Syndrome in children under 2 years is 0.0025 to 0.00815 mg/kg every 4 hours if needed. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Cystic Fibrosis in children under 2 years is 0.0025 to 0.00815 mg/kg every 4 hours if needed. A typical dosing of coated or uncoated digestive enzymes in combination with hyoscyamine is 2,500 U.S.P. units of lipase per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Prion Diseases in children under 2 years is 0.0025 to 0.00815 mg/kg every 4 hours if needed. A typical dosing of coated or uncoated digestive enzymes in combination with hyoscyamine is 2600 U.S.P. units of protease per kilogram three times per day for children under 2 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Autism, ADD, ADHD and other pervasive developmental disorders in children from 2 to 12 years is 0.0031 to 0.125 mg orally every 4 hours if needed. A typical dosing of coated or uncoated digestive enzymes in combination with hyoscyamine is 4,300 U.S.P. units of protease per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Familial Dysautonomia in children from 2 to 12 years is 0.0031 to 0.125 mg orally every 4 hours if needed. A typical dosing of coated or uncoated digestive enzymes in combination with hyoscyamine is 4,300 U.S.P. units of protease per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Guillain-Barre Syndrome in children from 2 to 12 years is 0.0031 to 0.125 mg orally every 4 hours if needed. A typical dosing of coated or uncoated digestive enzymes in combination with hyoscyamine is 4,300 U.S.P. units of protease per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of neuroblastoma in children from 2 to 12 years is 0.0031 to 0.125 mg orally every 4 hours if needed. A typical dosing of coated or uncoated digestive enzymes in combination with hyoscyamine is 4,300 U.S.P. units of protease per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in children from 2 to 12 years is 0.0031 to 0.125 mg orally every 4 hours if needed. A typical dosing of coated or uncoated digestive enzymes in combination with hyoscyamine is 4,300 U.S.P. units of protease per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of diabetic cardiovascular neuropathy in children from 2 to 12 years is 0.0031 to 0.125 mg orally every 4 hours if needed. A typical dosing of coated or uncoated digestive enzymes in combination with hyoscyamine is 4,300 U.S.P. units of protease per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Complex Regional Pain Syndrome in children from 2 to 12 years is 0.0031 to 0.125 mg orally every 4 hours if needed. A typical dosing of coated or uncoated digestive enzymes in combination with hyoscyamine is 4,300 U.S.P. units of protease per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Bipolar Disorder, Obsessive Compulsive Disorder or Oppositional Defiant Disorder in children from 2 to 12 years is 0.0031 to 0.125 mg orally every 4 hours if needed. A typical dosing of coated or uncoated digestive enzymes in combination with hyoscyamine is 4,300 U.S.P. units of protease per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of the symptoms of addiction in children from 2 to 12 years is 0.0031 to 0.125 mg orally every 4 hours if needed. A typical dosing of coated or uncoated digestive enzymes in combination with hyoscyamine is 4,300 U.S.P. units of protease per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of William's Syndrome in children from 2 to 12 years is 0.0031 to 0.125 mg every 4 hours if needed. A typical dosing of coated or uncoated digestive enzymes in combination with hyoscyamine is 4,300 U.S.P. units of protease per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Cystic Fibrosis in children from 2 to 12 years is 0.0031 to 0.125 mg every 4 hours if needed. A typical dosing of coated or uncoated digestive enzymes in combination with hyoscyamine is 2,500 U.S.P. units of lipase per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Prion Diseases in children from 2 to 12 years is 0.0025 to 0.125 mg every 4 hours if needed. A typical dosing of coated or uncoated digestive enzymes in combination with hyoscyamine is 4,300 U.S.P. units of protease per kilogram three times per day for children from 2 to 12 years of age. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Autism, ADD, ADHD and other pervasive developmental disorders in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 4,300 U.S.P. units of protease per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Familial Dysautonomia in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 4,300 U.S.P. units of protease per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Guillain-Barre Syndrome in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 4,300 U.S.P. units of protease per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of neuroblastoma in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 4,300 U.S.P. units of protease per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 4,300 U.S.P. units of protease per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of diabetic cardiovascular neuropathy in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 4,300 U.S.P. units of protease per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Complex Regional Pain Syndrome in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 4,300 U.S.P. units of protease per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Bipolar Disorder, Obsessive Compulsive Disorder or Oppositional Defiant Disorder in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 4,300 U.S.P. units of protease per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of the symptoms of addiction in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 4,300 U.S.P. units of protease per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of William's Syndrome in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 4,300 U.S.P. units of protease per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Cystic Fibrosis in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 2,500 U.S.P. units of lipase per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with hyoscyamine for the treatment of Prion Diseases in adults is 0.0125 to 0.25 mg in tablet or sublingual form, or 0.0375 to 0.75 mg twice daily in sustained-release capsule or tablet. A typical dosing of coated or uncoated digestive enzymes in combination with hyoscyamine is 4,300 U.S.P. units of protease per kilogram three times per day for adults. Dosing for both the enzyme composition or enzyme preparation and hyoscyamine may be from the minimal clinically proven safe and efficacious dosing to the maximal proven safe and efficacious dosage.

One example of a formulation of uncoated PEC in combination with atropine for the treatment of Autism, ADD, ADHD and other pervasive developmental disorders in children is 0.001 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of Familial Dysautonomia in children is 0.001 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of Guillain-Barre Syndrome in children is 0.001 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of neuroblastoma in children is 0.001 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in children is 0.001 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of diabetic cardiovascular neuropathy in children is 0.001 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of Complex Regional Pain Syndrome in children is 0.001 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of Bipolar Disorder, Obsessive Compulsive Disorder or Oppositional Defiant Disorder in children is 0.01 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of the symptoms of addiction in children is 0.01 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of William's Syndrome in children is 0.01 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of Cystic Fibrosis in children is 0.01 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 2,500 U.S.P. units of lipase per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of Prion Diseases in children is 0.01 to 0.03 mg/kg every 4 to 6 hours if needed in tablet or injection. A typical dose of atropine in combination with uncoated PEC might be 0.1 mg with each dose. A typical dosing of coated or uncoated digestive enzymes in combination with atropine is 4,300 U.S.P. units of protease per kilogram three times a day for children. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of Autism, ADD, ADHD and other pervasive developmental disorders in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 4,300 U.S.P. units of protease per kilogram three times a day for adults. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of Familial Dysautonomia in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 4,300 U.S.P. units of protease per kilogram three times a day for adults. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of Guillain-Barre Syndrome in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 4,300 U.S.P. units of protease per kilogram three times a day for adults. Dosing for both the enzyme composition or enzyme preparation and atropine may be from the minimal clinically proven safe and efficacious dosing to the maximal proven safe and efficacious dosage

One example of a formulation of uncoated PEC in combination with atropine for the treatment of neuroblastoma in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 4,300 U.S.P. units of protease per kilogram three times a day for adults. Dosing for both the enzyme composition or enzyme preparation and atropine may be from the minimal clinically proven safe and efficacious dosing to the maximal proven safe and efficacious dosage

One example of a formulation of uncoated PEC in combination with atropine for the treatment of other dysautonomias (including but not limited to the following: fetal fatal insomnia, Hereditary Sensory and Autonomic Neuropathy type III [HSAN], multiple system atrophy [Shy-Drager Syndrome], orthostatic intolerance syndrome including mitral valve prolapse, postural tachycardia syndrome [POTS], idiopathic hypovolemia, baroreflex failure, dopamine-B-hydroxylase deficiency, familial paraganglioma syndrome, tetrahydrobiopterin deficiency, aromatic-L-amino acid decarboxylase deficiency, Menke's disease, MAO deficiency states, Chagas disease, pure autonomic failure, syncope, hypertension, cardiovascular disease, and renal disease) in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 4,300 U.S.P. units of protease per kilogram three times a day for adults. Dosing for both the enzyme composition or enzyme preparation and atropine may be from the minimal clinically proven safe and efficacious dosing to the maximal proven safe and efficacious dosage

One example of a formulation of uncoated PEC in combination with atropine for the treatment of diabetic cardiovascular neuropathy in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 4,300 U.S.P. units of protease per kilogram three times a day for adults. Dosing for both the enzyme composition or enzyme preparation and atropine may be from the minimal clinically proven safe and efficacious dosing to the maximal proven safe and efficacious dosage

One example of a formulation of uncoated PEC in combination with atropine for the treatment of Complex Regional Pain Syndrome in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 4,300 U.S.P. units of protease per kilogram three times a day for adults. Dosing for both the enzyme composition or enzyme preparation and atropine may be from the minimal clinically proven safe and efficacious dosing to the maximal proven safe and efficacious dosage

One example of a formulation of uncoated PEC in combination with atropine for the treatment of Bipolar Disorder, Obsessive Compulsive Disorder or Oppositional Defiant Disorder in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 4,300 U.S.P. units of protease per kilogram three times a day for adults. Dosing for both the enzyme composition or enzyme preparation and atropine may be from the minimal clinically proven safe and efficacious dosing to the maximal proven safe and efficacious dosage

One example of a formulation of uncoated PEC in combination with atropine for the treatment of the symptoms of addiction in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 4,300 U.S.P. units of protease per kilogram three times a day for adults. Dosing for both the enzyme composition or enzyme preparation and atropine may be from the minimal clinically proven safe and efficacious dosing to the maximal proven safe and efficacious dosage

One example of a formulation of uncoated PEC in combination with atropine for the treatment of William's Syndrome in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 4,300 U.S.P. units of protease per kilogram three times a day for adults. Dosing for both the enzyme composition or enzyme preparation and atropine may be from the minimal clinically proven safe and efficacious dosing to the maximal proven safe and efficacious dosage.

One example of a formulation of uncoated PEC in combination with atropine for the treatment of Cystic Fibrosis in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 2,500 U.S.P. units of lipase per kilogram three times a day for adults. Dosing for both the enzyme composition or enzyme preparation and atropine may be from the minimal clinically proven safe and efficacious dosing to the maximal proven safe and efficacious dosage

One example of a formulation of uncoated PEC in combination with atropine for the treatment of Prion Diseases in adults is 0.04 to 0.6 mg every 4 to 6 hours if needed in tablet or injection. A typical dose of atropine in combination with uncoated PEC might be 0.4 mg with each dose. A typical dosing of coated or uncoated digestive enzymes in combination with atropine is 4,300 U.S.P. units of protease per kilogram three times a day for adults. Dosing for both the enzyme composition or enzyme preparation and atropine may be from the minimal clinically proven safe and efficacious dosing to the maximal proven safe and efficacious dosage.

The invention is further described byway of the following paragraphs:
1. A pharmaceutical composition comprising one or more digestive enzymes, wherein the one or more digestive enzymes are optionally coated, and one or more gastrointestinal modulators of acid, or pharmaceutically acceptable salts thereof.
2. The pharmaceutical composition of paragraph 1, wherein the coating is a lipid coating.
3. The pharmaceutical composition of paragraph 1, wherein the one or more digestive enzymes comprise pancreatic enzymes.
4. The pharmaceutical composition of paragraph 1, wherein the one or more digestive enzymes comprises at leastone amylase, lipase, and protease.
5. The pharmaceutical composition of paragraph 1, wherein the pharmaceutical composition is coated.

## Claims

1. A pharmaceutical composition for use in treating a subject in need thereof, comprising digestive enzymes and one or more agents which reduce stomach acid, or pharmaceutically acceptable salts thereof; wherein the digestive enzymes comprise an amylase, a lipase and a protease; wherein the digestive enzymes are coated with a controlled release lipid coating that provides release of the digestive enzymes in the proximal small intestines.

2. The pharmaceutical composition for use according to claim 1, wherein the lipid coating has a dissolution profile of between 30-90 minutes with 80% or greater release.

3. The pharmaceutical composition for use according to claim 1 or claim 2, wherein the lipid coating has a dissolution profile for release in the proximal small intestines at a pH of from 5.0 to 6.0.

4. The pharmaceutical composition for use according to any of claims 1 to 3, wherein the agent that reduces stomach acid is a proton pump inhibitor (PPI), optionally wherein said PPI is omeprazole, esomeprazole, a combination of omeprazole and sodium bicarbonate, lansoprazole, dexlansoprazole, rabeprazole, or pantoprazole.

5. The pharmaceutical composition for use according to any preceding claim, wherein the pharmaceutical composition is a tablet and the coating buffers the pharmaceutical composition to combat change in pH, optionally wherein the coating is buffered by potassium metaphosphate, potassium phosphate or monobasic sodium acetate.

6. The pharmaceutical composition for use according to any preceding claim, wherein the lipid coating comprises hydrogenated soybean oil.

7. The pharmaceutical composition for use according to any preceding claim, wherein the one or more digestive enzymes comprise pancreatic enzymes.

8. The pharmaceutical composition for use according to any preceding claim, wherein the amylase comprises from about 1,000 to about 15,000,000 U.S.P. units.

9. The pharmaceutical composition for use according to any preceding claim, wherein the protease comprises between 10,000 to 1,500,000 U.S.P. units.

10. The pharmaceutical composition for use according to any preceding claim, wherein the lipase comprises from about 1,880 to about 282,000 U.S.P. units.

11. The pharmaceutical composition for use according to any preceding claim, in the form of a tablet, further comprising one or more excipients wherein the one or more excipients comprise a binder, a glider, a lubricant, disintegrant, a sweetener, or a combination thereof.

12. The pharmaceutical composition for use according to any preceding claim, wherein the coating protects the digestive enzymes from destabilizing factors such as solvents, heat, light, moisture and environmental factors.

13. The pharmaceutical composition for use according to any preceding claim, for use in treating Autism, Attention Deficit Disorder (ADD), or Attention Deficit Hyperactive Disorder (ADHD), cystic fibrosis, Asperger syndrome, Rett syndrome, or Parkinson's Disease.

14. The pharmaceutical composition for use according to any preceding claim, wherein the digestive enzymes are coated in multiple layers of coatings.

15. The pharmaceutical composition for use according to any preceding claim, for use in treating behavioral effects of the conditions of claim 13.
